# EUROPEAN PATENT SPECIFICATION

(11) **EP 4 402 289 B1**
(45) Date of publication and mention of the grant of the patent: **01.04.2026**
(21) Application number: 22786633.2
(22) Date of filing: 15.09.2022
(51) Int. Cl.: C12Q 1/6883

(54) **METHODS OF USING DONOR-DERIVED CELL-FREE DNA TO DISTINGUISH ACUTE REJECTION AND OTHER CONDITIONS IN LIVER TRANSPLANT RECIPIENTS**
VERFAHREN ZUR VERWENDUNG VON SPENDER-ABGELEITETER ZELLFREIER DNA ZUR UNTERSCHEIDUNG VON AKUTER ABSTOSSUNG UND ANDEREN LEIDEN IN LEBERTRANSPLANTATEMPFÄNGERN
PROCÉDÉS D'UTILISATION D'ADN ACELLULAIRE ISSU D'UN DONNEUR POUR DISTINGUER UN REJET AIGU ET D'AUTRES ÉTATS CHEZ DES RECEVEURS DE GREFFE HÉPATIQUE

(30) Priority: 16.09.2021 US 202163245136 P
(43) Date of publication of application: 24.07.2024
(73) Proprietor: Northwestern University, Evanston, IL 60208 (US); Transplant Genomics, Inc., Framingham, Massachusetts 01702 (US)
(72) Inventor: KLEIBOEKER, Steve, Framingham, MA 01702 (US); SINHA, Rohita, Framingham, MA 01702 (US); MILLER, Mikaela, Framingham, MA 01702 (US); LEVITSKY, Josh, Evanston, IL 60208 (US); WEEMS, Juston, Framingham, MA 01702 (US); ABECASSIS, Michael M., Evanston, IL 60208 (US)
(74) Representative: Forresters IP LLP
(86) International application number: PCT/US2022/043709
(87) International publication number: WO 2023/043956

(56) References cited:
- WO-A1-2015/035367
- WO-A1-2020/010255
- US-A1- 2017 137 885
- BECK J ET AL: "Donor-Derived Cell-Free DNA Is a Novel Universal Biomarker for Allograft Rejection in Solid Organ Transplantation", TRANSPLANTATION PROCEEDINGS, vol. 47, no. 8, October 2015 (2015-10-01), pages 2400 - 2403, XP029318585, ISSN: 0041-1345, DOI: 10.1016/J.TRANSPROCEED.2015.08.035
- DE VLAMINCK IWIJN ET AL: "Circulating cell-free DNA enables noninvasive diagnosis of heart transplant rejection", SCIENCE TRANSLATIONAL MEDICINE, vol. 6, no. 241, 18 June 2014 (2014-06-18), pages 241ra77, XP008177554, ISSN: 1946-6242, [retrieved on 20140618], DOI: 10.1126/SCITRANSLMED.3007803
- LO Y D ET AL: "Presence of donor-specific DNA in plasma of kidney and liver-transplant recipients", THE LANCET, ELSEVIER, AMSTERDAM, NL, vol. 351, no. 9112, 2 May 1998 (1998-05-02), pages 1329 - 1330, XP004832243, ISSN: 0140-6736, DOI: 10.1016/S0140-6736(05)79055-3
- PATTAR SABRINA K ET AL: "Circulating nucleic acids as biomarkers for allograft injury after solid organ transplantation: current state-of-the-art", TRANSPLANT RESEARCH AND RISK MANAGEMENT, vol. Volume 11, 1 November 2019 (2019-11-01), pages 17 - 27, XP093010175, Retrieved from the Internet <URL:https://pdfs.semanticscholar.org/8091/6e1d2a566b2fbda02463286c9b06369cdf0b.pdf> DOI: 10.2147/TRRM.S204233
- PAUL M. K. GORDON ET AL: "An Algorithm Measuring Donor Cell-Free DNA in Plasma of Cellular and Solid Organ Transplant Recipients That Does Not Require Donor or Recipient Genotyping", FRONTIERS IN CARDIOVASCULAR MEDICINE, vol. 3, no. 33, 22 September 2016 (2016-09-22), pages 1 - 10, XP055440493, DOI: 10.3389/fcvm.2016.00033
- LEVITSKY JOSH ET AL: "Donor-derived cell-free DNA levels predict graft injury in liver transplant recipients", AMERICAN JOURNAL OF TRANSPLANTATION, vol. 22, no. 2, 24 February 2022 (2022-02-24), DK, pages 532 - 540, XP093010168, ISSN: 1600-6135, Retrieved from the Internet <URL:https://onlinelibrary.wiley.com/doi/full-xml/10.1111/ajt.16835> DOI: 10.1111/ajt.16835

## Description

### FIELD

Described herein are methods, compositions, and systems useful for detecting transplant rejection and associated abnormal conditions in liver transplant recipients involving an assessment of donor-derived cell-free nucleic acids (dd-cfNA) such as donor-derived cell-free DNA (dd-cfDNA).

### BACKGROUND

Despite the availability of efficacious immunosuppression, up to a third of liver transplant recipients still experience acute rejection (AR). Clinicians may prescribe an immunosuppressant regimen to prevent and treat AR. Preventing AR is particularly mportant as AR has been shown to reduce both graft and patient survival in liver transplant recipients. However, complications associated with such immunosuppressant regimens can negatively affect their overall benefit. Clinicians do not currently possess sufficient tools to be able to restrict the use of immunosuppressant regimens to those patients likely to experience AR beyond testing immunosuppression levels and performing liver function tests (LFTs), which are suboptimal.

As a result of these factors, there is a strong interest in immunosuppressive minimization protocols to both prevent and treat immunosuppressive complications, including nephrotoxicity due to calcineurin inhibitor (CNI) therapy among others. Yet without specific tests indicating the onset of immune activation, clinicians currently perform arbitrary 'trial and error' immunosuppressive reductions which can trigger rejection and alternatively the need for higher prolonged immunosuppressant dosing and worse overall outcomes.

Non-invasive biomarkers that can detect immune activation and imminent graft dysfunction in the context of immunosuppressive tapering would be a welcome addition to liver transplant recipient management.

### SUMMARY

The present invention is defined by the appended claims.

There is a need for improved methods, compositions, and systems for assessing liver transplant rejection in liver transplant recipients, particularly in a minimally invasive manner. As shedding of donor- derived cell- free DNA (dd- cfDNA) into the recipient circulation has been documented following liver transplantation, methods using this phenomenon offer the potential to assess liver transplant rejection and associated conditions in such a minimally invasive manner.

In some aspects, the present disclosure provides for a method of distinguishing non-rejection (TX) from one or both of acute rejection (AR) and acute dysfunction non-rejection (ADNR) in a liver transplant recipient, the method comprising: (a) obtaining a sample from a liver transplant recipient; (b) obtaining cell-free DNA (cfDNA) from the sample; (c) determining a level of donor derived cell-free DNA (dd-cfDNA) in the cfDNA; and (d) distinguishing non-rejection from one or both of AR and ADNR in the recipient by comparing the level of dd-cfDNA to a pre-determined threshold value. In some cases, the level of dd-cfDNA is determined by sequencing the cfDNA, and inputting into a computer-implemented algorithm (i) cfDNA sequence information at a plurality of genetic loci, and (ii) genotype information from the recipient, in the absence of genotype information from the donor, wherein the algorithm identifies dd-cfDNA sequences at the plurality of genetic loci by identifying a pattern of cfDNA sequences at the loci that differ from the cfDNA sequences of the recipient but that correlate with a pattern of human DNA sequences found in a database of human genomes. In some cases, determining the level of dd-cfDNA comprises analyzing cfDNA sequences of at least 1000, at least 2000, at least 5000, at least 10,000, at least 20,000, at least 100,000, at least 500,000, at least 1,000,000, at least 2,000,000, or at least 2,500,000 genetic loci, such as SNPs. In some cases, the sample is a blood, serum, plasma, or urine sample. In some cases, the sample is a blood sample. In some cases, plasma is separated from the blood sample. In some cases, peripheral blood mononuclear cells (PBMCs) are isolated from said blood sample. I some such cases, genotype information from the recipient is obtained from the PBMCs.

In some methods herein, the liver transplant recipient has a normal serum alanine aminotransferase concentration, such as of less than or equal to 60 IU/L for a male recipient or less than or equal to 36 IU/L for a female recipient, or the serum alanine aminotransferase concentration has not doubled compared to a baseline concentration determined after transplantation. In some cases, the liver transplant recipient has a normal serum aspartate aminotransferase concentration, such as below 40 IU/L, or the serum aspartate aminotransferase concentration has not doubled compared to a baseline concentration determined after transplantation. In some cases, the liver transplant recipient has a normal serum alkaline phosphatase level, such as below 200 U/L, or the serum alkaline phosphatase level has not doubled compared to a baseline concentration determined after transplantation. In some cases, the liver transplant recipient has a normal total bilirubin concentration, such as less than or equal to 1.5 mg/dL, or the total bilirubin concentration has not doubled compared to a baseline concentration determined after transplantation. In some cases, the liver transplant recipient has a normal direct bilirubin concentration, such as below 0.5 mg/dL, or the direct bilirubin concentration has not doubled compared to a baseline concentration determined after transplantation.

In some cases, determining the level of the dd-cfDNA comprises sequencing the cfDNA by whole genome sequencing, or by next generation sequencing, such as unbiased next generation sequencing, or by other methods disclosed herein. In some cases, the recipient is indicated as having a likelihood of AR by a level of dd-cfDNA that is greater than or equal to a pre-determined threshold of from 18% to 25%, from 18% to 23%, from 19% to 22%, from 19% to 21%, from 20% to 22%, from 20% to 21%, from 20.2% to 20.6%, or a pre-determined threshold of 18%, 19%, 20%, 20.2%, 20.4%, 20.6%, 20.8%, 21%, 22%, 23%, or 25%. In some such cases, the recipient is indicated as having a likelihood of AR by a level of dd-cfDNA that is greater than or equal to a pre-determined threshold of 20.4% . In some cases, the recipient is indicated as having AR or ADNR by a level of dd-cfDNA that is greater than or equal to a pre-determined threshold of from 10% to 17%, from 13% to 17%, from 13% to 15%, from 15% to 17%, from 14% to 17%, from 149% to 16%, from 14% to 15%, from 15% to 16%, from 14.5% to 15.5%, or a pre-determined threshold of 10%, 11%, 12%, 13%, 14%, 14.5%, 15%, 15.5%, 16%, or 17%. In some such cases, the recipient is indicated as having a likelihood of AR or ADNR by a level of dd-cfDNA that is greater than or equal to a pre-determined threshold of 15.0%. In some cases, the recipient is indicated as having a likelihood of non-rejection by a level of dd-cfDNA that is less than a pre-determined threshold of from 4% to 7%, from 4% to 6%, from 5% to 7%, from 4.5% to 6.5%, from 4.5% to 5.5%, from 5% to 6%, from 5% to 5.5%, or less than a pre-determined threshold value of 49%, 4.5%, 5%, 5.1%, 5.2%, 5.3%, 5.4%, 5.5%, 6%, 6.5%, or 7%. In some such cases, the recipient is indicated as having a likelihood of non-rejection by a level of dd-cfDNA that is less than a pre-determined threshold of 5.3%. In some cases, the recipient is indicated as having a likelihood of ADNR by a level of dd-cfDNA that is greater than or equal to a pre-determined threshold of 5.3% but less than a pre-determined threshold of 15.0%.

In some cases, methods herein, such as those above, are performed at least one month, at least two months, at least three months, at least six months, or at least one year after transplantation. In some cases, methods herein further comprise determining one or more of: alanine aminotransferase concentration, aspartate aminotransferase concentration, alkaline phosphatase concentration, direct bilirubin concentration, or bilirubin concentration in the sample. In some cases, methods herein further comprise conducting a liver biopsy on the recipient. In some cases, methods herein further comprise (e) providing a report summarizing the recipient's level of dd-cfDNA, and optionally one or more potential treatments for the recipient. In some cases, methods herein further comprise (f) selecting a treatment for the recipient based on the recipient's level of dd-cfDNA.

Additional objects and advantages will be set forth in part in the description which follows, and in part will be understood from the description, or may be learned by practice. The objects and advantages will be realized and attained by means of the elements and combinations particularly pointed out in the appended claims. It is to be understood that both the foregoing general description and the following detailed description are exemplary and explanatory only and are not restrictive of the claims.

The accompanying drawings, which are incorporated in and constitute a part of this specification, illustrate certain embodiments and together with the description, serve to further explain certain principles described herein.

### BRIEF DESCRIPTION OF THE DRAWINGS

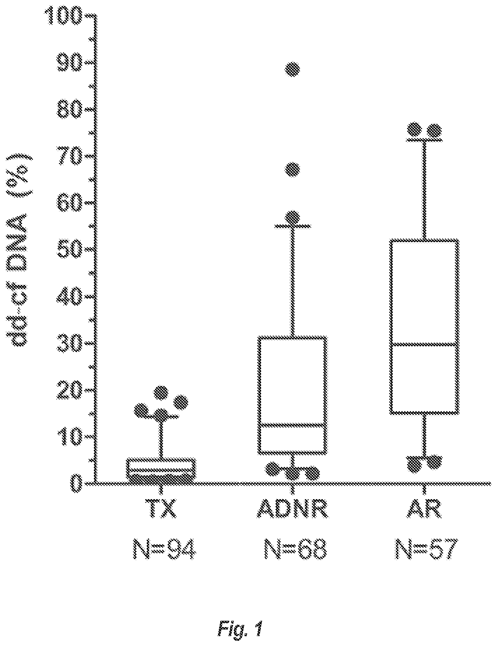

**Figure 1 (FIG. 1****)** depicts a box-and-whisker plot of plasma dd-cfDNA values for TX, ADNR, and AR patients. Boxes represent median and inter-quartile ranges and whiskers show 5th-9th percentile. Dots represent outlier samples. N denotes number of contributing samples. * denotes p<0.000 I significance by two-tailed Mann-Whitney test.

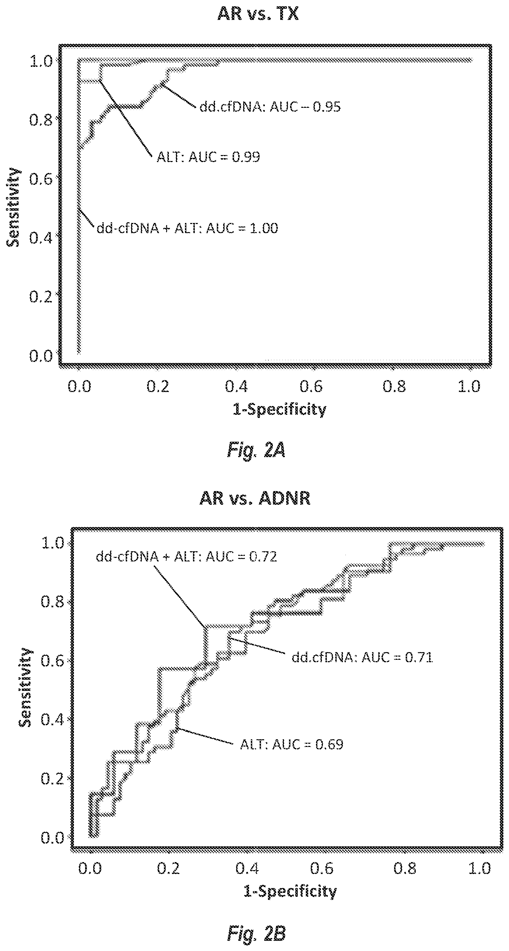

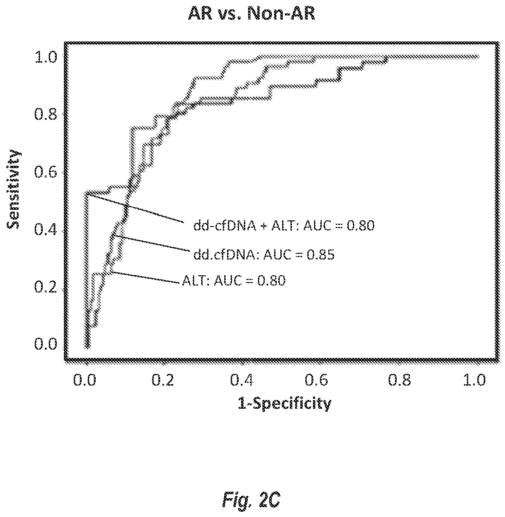

**Figures 2A**, **2B****, and** **2C** **(****FIG. 2A, 2B****,** **2C****)** depict receiver operator characteristic (ROC) curve analyses (as graphs) of dd-cfDNA training set values in (Fig. 2A) AR versus TX, (Fig. 2B) AR versus ADNR, (Fig. 2C) AR versus Non-AR. Each graph shows ROC curve and area under the curve (AUC) comparisons between dd-cfDNA alone, ALT alone, and ALT in combination with dd-cfDNA.

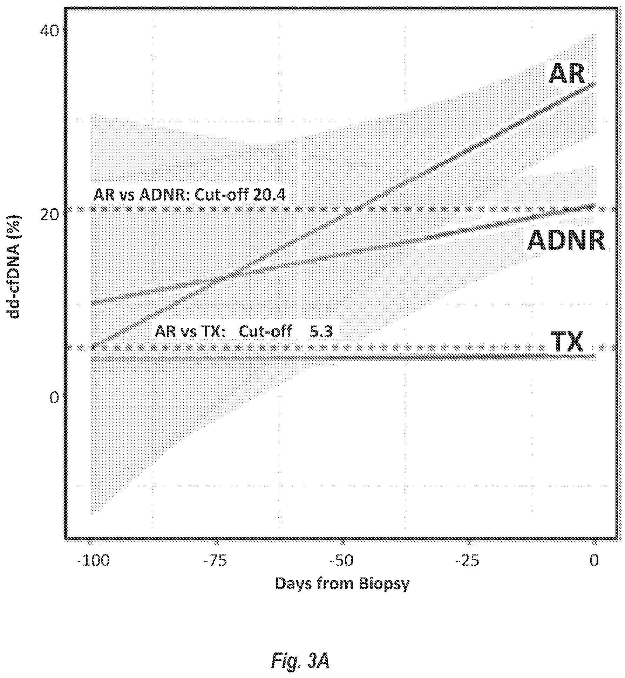

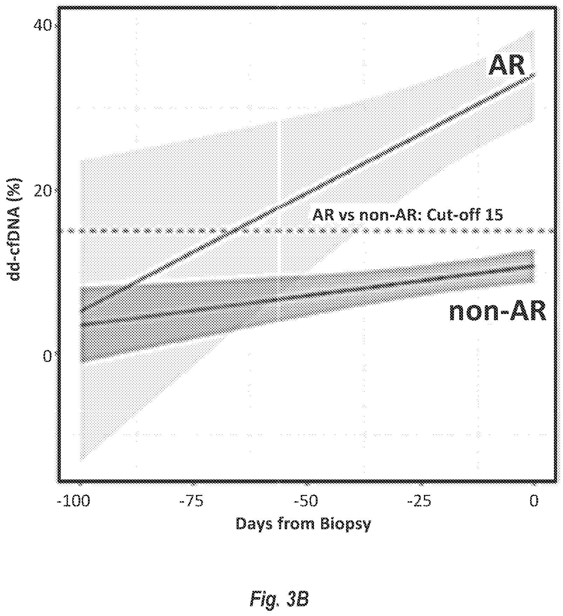

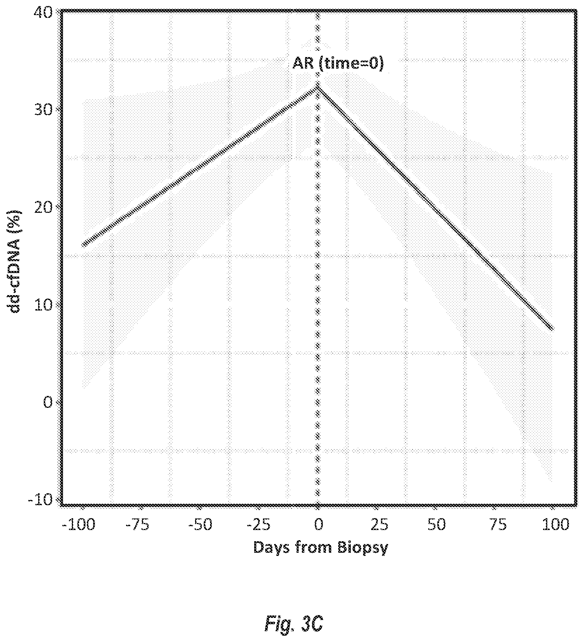

**Figures 3A****,** **3B****, and** **3C** **(****FIG. 3A****,** **3B****,** **3C****)** show serial changes in dd-cfDNA over time in (Fig. 3A) Pre-AR, Pre-ADNR, and Pre-TX, (Fig. 3B) Pre-AR and Pre-Non-AR, (Fig. 3C) Pre-AR to Post-AR. Depicted are graphs visually displaying the dd-cfDNA trajectories 100 days before and after biopsy or TX time point (time=0). The shaded region shows the 95% confidence interval for the LME model. The cut-offs established in the diagnostic analysis (see Table 2) are shown as dashed lines differentiating classes.

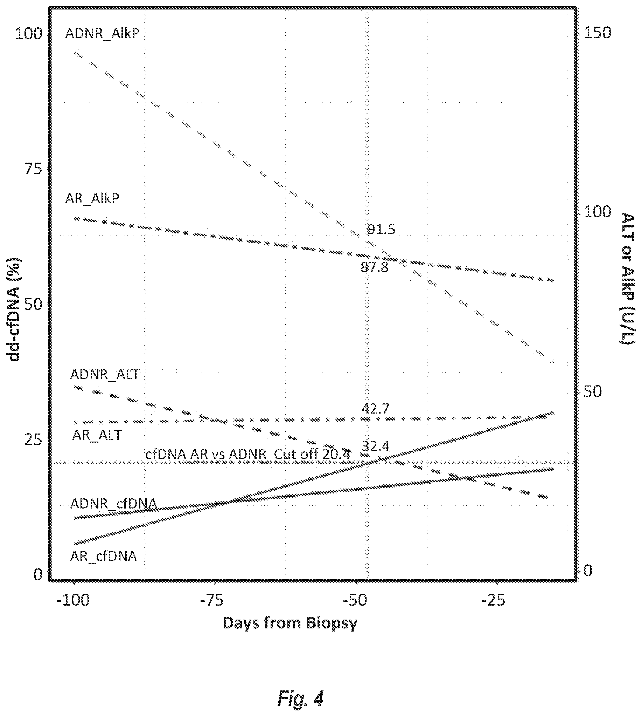

**Figure 4 (FIG. 4****)** shows serial changes in dd-cfDNA, ALT, and AlkP from day -100 to -14 prior to AR and ADNR graft injury diagnoses. Shown is a graph of line plots generated by fitting LME models. The horizontal dotted line marks the 20.4% AR versus ADNR cut-off established in the diagnostic dd-cfDNA analysis and the vertical dotted line shows the ALT and AlkP values at the cut-off time point (day -48).

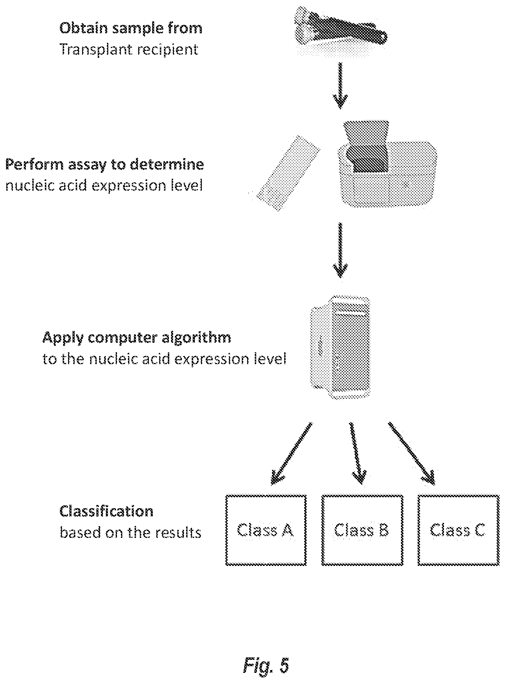

**Figure 5 (FIG. 5****)** depicts an schematic showing how samples can be classified according to methods described herein.

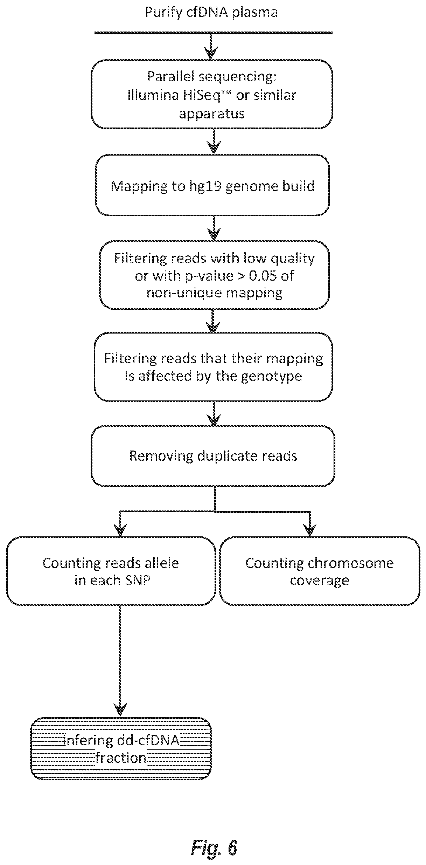

**Figure 6 (FIG. 6****)** shows an example cfDNA sequencing and genotyping data processing pipeline of one potential method of cfDNA sequencing and genotyping as described herein, which relies on genotype data from a recipient. Illustration of the pipeline used to retrieve allele counts in cfDNA fragments for each recipient-genotyped SNP from the raw cfDNA sequencing and genotyping measurements is shown.

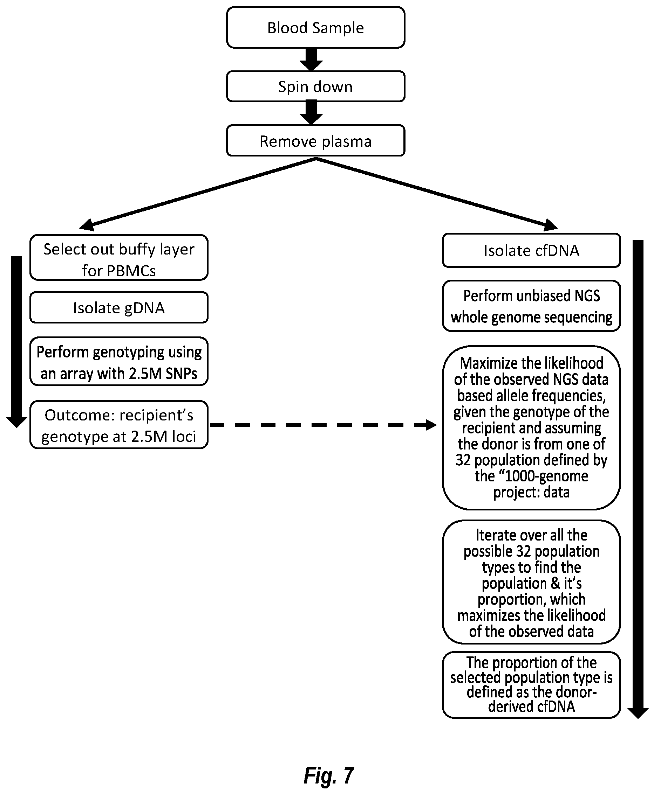

**Figure 7 (FIG. 7****)** shows a schematic example of how cfDNA may be isolated from a blood sample and how dd-cfDNA level may be detennined.

### DETAILED DESCRIPTION OF THE INVENTION

### Definitions

Unless defined otherwise, all technical and scientific terms used herein have the same meaning as commonly understood by those of ordinary skill in the art to which this invention pertains. In addition, the following definitions are provided to assist the reader in the practice of the invention.

The term "or" as used herein and throughout the disclosure, is intended as an inclusive "or", meaning "and/or" unless the context expressly indicates otherwise.

The terms "a" or "the" as used herein and throughout the disclosure are intended to encompass both singular and plural, i.e., to mean "at least one," unless the context expressly indicates otherwise.

The terms "transplantation" or a "transplant" generally refer to the transfer of tissues, cells, or a solid organ (i.e., a liver) from a donor individual into a recipient individual. A donor and recipient may or may not be from the same species. Thus, for example, a human recipient may receive a solid organ from a non-human animal in some embodiments. An "allograft" further indicates a transfer of tissues, cells, or a solid organ between different individuals of the same species. In contrast, if the donor and recipient are the same individual, the graft is referred to as an "autograft."

A "recipient" generally refers to an individual receiving a transplant, allograft, or autograft. A "recipient" herein is a human, unless expressly stated otherwise (i.e., a murine recipient or the like). The terms "individual," "subject," or "patient" in the context of transplantation or medical treatment generally refer interchangeably to a human receiving such a transplantation or other medical treatment, e.g., a recipient of a transplant or of other medical treatment, unless the context clearly indicates a different meaning.

As used herein, a recipient that does not have rejection, or that shows "non-rejection," or is negative for rejection, or the like, which may also be abbreviated "TX" herein, generally signifies that the recipient does not exhibit symptoms or test results indicating organ dysfunction or rejection. Accordingly, in such recipients the transplant is considered a normal functioning transplant. A "non-rejection" or "TX" patient can have normal histology on a surveillance biopsy (e.g. no evidence of rejection). In some embodiments a TX subject may display a serum alkaline phosphatase level below 200 IU/L; serum alanine aminotransferase concentration less than or equal to 60 IU/L, for a male recipient or less than or equal to 36 IU/L for a female recipient; serum aspartate aminotransferase concentration below 40 IU/L; direct bilirubin concentration of less than 0.5 mg/dL; and/or total bilirubin concentration less than or equal to 1.5 mg/dL. In contrast, a recipient experiencing acute rejection (AR) or acute disfunction non-rejection (ADNR) (also termed "non-TX" herein) can be observed either clinically or subclinically, for example, such as via biomarker tests herein or via histology.

"Acute rejection (AR)" generally refers to a condition that can occur when transplanted tissue is rejected by the recipient's immune system, which damages or destroys the transplanted tissue unless immunosuppression is achieved. T-cells, B-cells and other immune cells as well as possibly antibodies of the recipient may cause the graft cells to lyse or produce cytokines that recruit other inflammatory cells, eventually causing necrosis of allograft tissue. In some instances, AR can be diagnosed by a biopsy of the transplanted organ. In the case of liver transplant recipients, AR may be assessed in severity according to the Banff schema for liver transplant rejection as described in e.g., Ormonde et al. (Liver Transpl Surg. 1999 Jul;5(4):261-8).

"Acute dysfunction no rejection (ADNR)" or "acute dysfunction without rejection" or "acute dysfunction non-rejection" phenotype, in which the subject shows symptoms of or biomarkers associated with dysfunction of the transplanted organ, but does not show symptoms or biomarkers associated with rejection. In some cases, a subject, such as a liver transplant subject, may show evidence of elevated serum alanine aminotransferase concentration, senini aspartate aminotransferase concentration, serum alkaline phosphatase level, direct bilirubin concentration, and/or total bilirubin concentration. In some embodiments, ADNR comprises biliary obstruction, cholestasis, steatosis, drug-induced liver injury, or infection.

The term "liver function test" or "LFT" refers to a blood test that measures levels of certain molecules in blood (i.e., whole blood or serum). In some embodiments, LFT measure levels of certain enzymes and proteins in a sample. For example, an LFT can include measuring the levels of alanine aminotransferase (ALT), aspartate aminotransferase (AST), alkaline phosphatase (ALP), direct bilirubin, and/or total bilirubin. In some embodiments, an LFT can include measurement of albumin and total protein, gamma-glutamyltransferase (GGT), L-lactate dehydrogenase (LD), and/or prothrombin time (PT).

A "biopsy" generally refers to a specimen obtained from a living patient for diagnostic or prognostic evaluation. A "surveillance biopsy" for example may be performed following a transplant to look for evidence of rejection or non-rejection. Liver biopsies can be obtained with a needle.

As used herein, in performing the methods, "obtaining a sample" includes obtaining a sample directly or indirectly. In some embodiments, the sample is taken from the subject by the same party (e.g. a testing laboratory) that subsequently acquires biomarker data from the sample. In some embodiments, the sample is received (e.g. by a testing laboratory) from another entity that collected it from the subject (e.g. a physician, nurse, phlebotomist, or medical caregiver). In some embodiments, the sample is taken from the subject by a medical professional under direction of a separate entity (e.g. a testing laboratory) and subsequently provided to said entity (e.g. the testing laboratory). In some embodiments, the sample is taken by the subject or the subject's caregiver at home and subsequently provided to the party that acquires biomarker data from the sample (e.g. a testing laboratory). In some cases, the sample is obtained directly from the subject (e.g., obtained via a self sampling procedure). As used herein, when a method herein is said to be conducted at a particular time, such as a specific time after transplantation (e.g., 1week, 1month, etc. following transplantation), where there is a delay between the time that the sample was taken from the recipient and when the dd-cfDNA data were obtained, the method is said to be conducted at the time that the sample was taken from the recipient, since the results reflect the state of the recipient at that point in time.

The term "cell-free nucleic acid" or "cfNA" refers to nucleic acids that are detectable in biological samples such as blood or other samples herein without performing cell lysis or other procedures to extract nucleic acids from cells. In some embodiments, cfNA is DNA, and thus, cell-free DNA" or "cfDNA."

The terms "donor-derived cell-free nucleic acid" or "dd-cfNA" or "donor-derived cell-free DNA" or "dd-cfDNA" refer to the portion of cfNA or of DNA that is estimated to come from the donated tissue or organ. The "level" or "portion" of dd-cfDNA in a cfDNA sample, for example, can be expressed as a percentage of the total or an equivalent fraction of the total (such as, e.g., 10% or 0.1 or 1/10 or the like).

The term "treatment," for example, for a transplant recipient, includes medical management strategies such as active surveillance, which may include diagnostic or biopsy assays to assess likelihood of rejection, as well as therapeutic treatment, for example, with drugs intended to suppress rejection or promote functioning of the transplanted organ, such as immunosuppressants. Further discussion of treatments is provided below.

An "immunosuppressant drug" or "immunosuppressive drug" refers to a class of drugs that is intended to reduce the strength of the body's immune system.

The terms "immunosuppressant drug regimen" or "immunosuppressant treatment regimen", as used herein, refers to a set of at least one drug with immunosuppressant activity which is administered to a patient on an ongoing basis to treat or prevent allograft rejection. Immunosuppressant drug regimens may include, but are not limited to, an "induction" regimen (which is administered to a patient immediately before and optionally immediately after transplantation, see e.g., Charlton et al. Transplantation. 2018 May;102(5):727-743.), a maintenance regimen, a breakout regimen, or a combination thereof.

The term "genotype information" includes sufficient sequencing of the recipient's genome to cover specific loci and does not necessarily refer to sequencing the entire genome. In some embodiments, the "genotype information" refers to HLA-C genotype.

"Whole genome sequencing" includes sequencing the entire genome of a patient providing complete genomic information. In some embodiments, whole genome sequencing can be conducted by non-biased next generation sequencing.

A "likelihood" of a particular clinical response such as AR and ADNR may be obtained in certain methods herein. For example, certain biomarker tests can indicate whether a recipient is likely to have AR or ADNR, or whether a recipient is likely to be non-TX.

The term "significantly different" in the methods herein, i.e., in referring differences in dd-cfDNA levels in rejection vs. non-rejection subjects, means statistically significantly different, such as through a T-test and an associated P value that indicates statistical significance. Similarly, if other dd-cfDNA levels that are "not significantly different," the changes are not statistically significantly different. Statistical significance generally refers to p < 0.05 or < 0.01 or < 0.001 or even < 0.0001 level.

Additional definitions of particular terms are provided in the sections that follow.

### Methods of Distinguishing Rejection from Non-rejection

The present disclosure relates to methods capable of distinguishing rejection non-rejection from one or both of acute rejection (AR) and acute dysfunction non-rejection (ADNR) in a liver transplant recipient that, in some embodiments, comprise determination of the level of donor-derived, cell free nucleic acid (dd-cfNA), such as donor-derived, cell-free DNA (dd-cfDNA) in a sample from the recipient and comparing the level of dd-cfNA or dd-cfDNA to a pre-determined threshold value indicating whether or not the recipient does not have rejection (non-rejection or TX) or is likely to have AR or ADNR. Certain methods herein comprise: obtaining a sample from the liver transplant recipient; obtaining cell-free nucleic acid or cell-free DNA (cfNA or of BNA) from the sample; determining the level of donor-derived cell-free nucleic acid or donor-derived cell-free DNA (dd-cfN-A or dd-cfDNA) in the cfNA or cfDNA; and distinguishing non-rejection (i.e., TX) from one or both of AR and ADNR in the recipient by comparing the level of dd-cfNA or dd-cfDNA to a pre-determined threshold value. In some embodiments, the pre-determined threshold value is from 4% to 25%.

In some embodiments, AR is indicated by a level of dd-cfDNA that is greater than or equal to a pre-determined threshold of from 18% to 25%, from 18% to 23%, from 19%) to 22%, from 19% to 21%, from 20% to 22%, from 20% to 21%, from 20.2% to 20.6%, or a pre-determined threshold of 18%, 19%, 20%, 20.2%, 20.4%, 20.6%, 20.8%, 21%, 22%, 23%, or 25%. In some embodiments, the pre-determined threshold is 20.4%.

In some embodiments, AR or ADNR is indicated by a level of dd-cfDNA that is greater than or equal to a pre-determined threshold of from 10% to 17%, from 13% to 17%, from 13% to 15%, from 15% to 17%, from 14% to 17%, from 14% to 16%, from 14% to 15%, from 15% to 16%, from 14.5% to 15.5%. or a pre-determined threshold of 10%, 11%, 12%, 13%, 14%, 14.5%, 15%, 15.5%, 16%, or 17%. In some embodiments, the pre-determined threshold is 15.0%.

In some embodiments, non-rejection is indicated by a level of dd-efDNA that is less than a pre-determined threshold of from 4%) to 7%, from 4% to 6%, from 5% to 7%, from 4.5% to 6.5%, from 4.5% to 5.5%, from 5% to 6%, from 5% to 5.5%, or less than a pre-determined threshold value of 4%, 4.5%, 5%, 5.1%, 5.2%, 5.3%, 5.4%, 5.5%, 6%, 6.5%, or 7%. In some embodiments, the pre-determined threshold is 5.3%.

In some embodiments, presence of ADNR can be determined by a threshold that is less than a pre-determined threshold of from 10% to 17%, from 13% to 17%, from 13% to 15%, from 15% to 17%, from 14% to 17%, from 14% to 16%, from 14% to 15%, from 15% to 16%, from 14.5% to 15.5%, or a pre-determined threshold of 10%, 11%, 12%, 13%, 14%), 14.5%, 15%, *15.5%,* 16%, or 17%, or 15.0%, but that is also greater than a pre-determined of from 4% to 7%, from 4% to 6%, from 5% to 7%, from 4.5% to 6.5%, from 4.5% to 5.5%, from 5% to 6%, from 5% to 5.5%, or less than a pre-determined threshold value of 4%, 4.5%, 5%, 5.1%, 5.2%, 5.3%, 5.4%, 5.5%, 6%, 6.5%, or 7%, such as 5.3%.

In various embodiments, recipients have undergone a liver transplant within 6 hours, 12 hours, 1day, 2 days, 3 days, 4 days, 5 days, 10 days, 15 days, 20 days, 25 days, 1month, 2 months, 3 months, 4 months, 5 months, 7 months, 9 months, 11 months, 1 year, 2 years, 4 years, 5 years, 10 years, 15 years, 20 years or longer of prior to being assessed by a method herein. In some cases, methods herein are performed every 1 month, 2 months, 3 months, 6 months, or year following a transplant procedure, for example. In some cases, they are performed every 2 months. In some cases, every 3 months. In some cases, every 6 months. In some cases, the frequency depends on the test results. Thus, for example, in some cases methods herein may be performed with increased frequency if one or both results is positive, for instance, if a treatment is subsequently adjusted.

In some cases, methods herein also include determining one or more of: alanine aminotransferase concentration, aspartate aminotransferase concentration, alkaline phosphatase concentration, direct bilirubin concentration, or bilirubin concentration in the sample. As discussed below, in other cases, methods herein are performed on patients with particular liver function test values. In some cases, the method further comprises conducting a liver biopsy on the recipient. In other cases, the method does not comprise performing a liver biopsy test, for example, in a recipient with normal LFT values. For example, the method may be used as a non-invasive alternative to performing a liver biopsy test. In some cases, the methods herein may be performed prior to determining a therapeutic regimen for preventing or reducing symptoms of AR or ADNR in a recipient. In other cases, a recipient is already receiving an immunosuppressant drug regimen. In such cases, the method herein may be performed in part to determine whether such treatment regimen can be altered, for example, to reduce the dosage or frequency of the immunosuppressant. In some cases, methods herein further comprise a step (e) of providing a report summarizing the recipient's level of dd-cfDNA, and optionally one or more potential treatments for the recipient. For example, in some cases, a score from the methods herein, such as a percent dd-ctFNA, may be accompanied with information on treatment options for other patients with similar dd-cfDNA values and LFT results. In some cases, the methods further comprise (f) selecting a treatment for the recipient based on the recipient's level of dd-cfDNA.

Additional aspects of the methods herein are addressed, for example, in the sections that follow and in the exemplary embodiments provided further below.

### Exemplary Samples

The methods in some embodiments may be conducted on a single sample from the recipient, for instance, a blood, serum, plasma, or urine sample, or a sample obtained by a non-invasive, minimally-invasive, or invasive procedure as discussed below from which cfNA can be obtained. In some embodiments, the dd-cfNA information is obtained from a single sample from the recipient. Such a "single sample" means herein a sample that is obtained from the recipient at one time, such as during one blood draw or phlebotomy appointment or during one other diagnostic or medical appointment. Accordingly, the "single sample" is not required to be present in the same sample container, but instead is merely drawn from the patient at the same time, during the context of one diagnostic or medical appointment.

In some embodiments, the sample is obtained from a non-invasive procedure, such as a throat swab, buccal swab, bronchial lavage, urine collection, skin or epidermal scraping, feces collection, menses collection, or semen collection. In other cases, a minimally-invasive procedure may be used such as a blood draw, e.g., by venipucture methods. In other cases, a sample may be obtained by an invasive procedure such as a biopsy, alveolar or pulmonary lavage, or needle aspiration. In other cases, a sample of capillary blood could be either self-collected or collected by a caregiver or healthcare provider.

In some embodiments, the sample is a blood, serum, or plasma sample. A "blood" sample, herein refers to whole blood or fractions thereof, including plasma, lymphocytes, peripheral blood lymphocytes (PBLs), peripheral blood mononuclear cells (PBMCs), serum, T cells, B Cells, CD3 cells, CD8 cells, CD4 cells, or other immune cells. In some embodiments, it is a whole blood sample. In some embodiments, PBMCs are isolated from the blood sample. In some embodiments, plasma is separated from the blood sample. In some embodiments, both plasma and PBMCs are isolated from the blood sample. In some embodiments, the sample is plasma.

Other samples that can be analyzed include urine, feces, saliva, and or other samples from which cfNA may be obtained. However, a sample may be any material containing tissues, cells, nucleic acids, genes, gene fragments, expression products, polypeptides, exosomes, gene expression products, or gene expression product fragments of a transplant recipient to be tested.

In some embodiments, a whole blood sample drawn from the recipient for analysis according to the methods herein may be, for example, 10 mL or less, 8 mL or less, 7 mL or less, 6 mL or less, or 5 mL or less. In some embodiments, a blood sample may be 6 mL or less. A blood sample may be obtained by a minimally-invasive method such as a blood draw or fingerstick or dried blood spot (DBS). The sample may be obtained by venipuncture or fingerstick via lancet device or via a capillary blood collection device. Some or all of a sample obtained from a recipient may then be used in the methods. In some embodiments, multiple samples may be obtained by the methods herein to ensure a sufficient amount of biological material. In some cases, methods herein may be performed on more than one recipient's sample, i.e., on pooled samples, then deconvoluted to determine whether any of the samples indicate rejection.

In some cases, the methods include further steps involving obtaining or analyzing a biopsy sample (e.g., liver biopsy). In cases where biopsies are obtained, the biopsies may be processed included by placing the samples in a vessel (e.g., tube, vial, microfuge tube, etc.) and storing them at a specific location such as a biorepository. The samples may also be processed by treatment with a specific agent, such as an agent that prevents nucleic acid degradation or deterioration, particularly an agent that protects RNA (e.g., RNALater) or DNA. In some cases, biopsies are subjected to histologic analysis including staining (e.g., hematoxylin and eosin (H&E) stain) probing (e.g., a probe attached to a dye, a probe attached to a fluorescent label). In some cases, the staining (e.g., H&E) may be analyzed by a blinded physician such as a blinded pathologist, or at least two blinded pathologists. In some cases, a histologic diagnosis is reconciled with laboratory data and clinical courses by one or more clinicians (e.g., at least two clinicians) prior to biomarker analyses.

### Exemplary Liver Transplant Recipients

Liver transplant recipients herein are humans unless specifically stated to be a different animal, such as a non-human primate (e.g., ape, monkey, chimpanzee), a domestic animal such as a cat, dog, or rabbit, or a livestock animal such as a goat, horse, cow, pig, or sheep, or a laboratory animal such as a rodent, mouse, SCID mouse, rat, guinea pig, etc.

The donor organ, tissue, or cells may be derived from a subject who has certain similarities or compatibilities with the recipient subject. For example, the donor organ, tissue, or cells may be derived from a donor subject who is age-matched, ethnicity-matched, gender-matched, blood-type compatible, or HLA-type compatible with the recipient subject. In some circumstances, the donor organ, tissue, or cells may be derived from a donor subject that has one or more mismatches in age, ethnicity, gender, blood-type, or HLA markers with the transplant recipient due to organ availability. The organ may be derived from a living or deceased donor.

In some embodiments, the recipient is undergoing a treatment regimen, or being evaluated for a treatment regimen, such as immunosuppressive therapy, to inhibit rejection or to reduce at least one symptom of rejection. However, in some instances, the recipient is not undergoing a treatment regimen such as immunosuppressant therapy. In some embodiments, the subject is receiving a standard of care immunosuppressant therapy regimen for liver transplant. In some embodiments, the recipient has not received a biopsy, such as a surveillance biopsy prior to assessment via a method herein.

In some embodiments, if the result of the method indicates that the recipient has AR or ADNR, the method comprises issuing a report to the recipient or the recipient's physician indicating possible treatment regimens. In some embodiments, the recipient has received at least one immunosuppressive drug, and, if the result of the method indicates that the recipient has AR or ADNR, the method comprises increasing the frequency or dosage of the at least one immunosuppressant drug, administering a further immunosuppressant drug, or administering a different immunosuppressive drug to the recipient. Alternatively, if the method indicates that the recipient is TX, the method might further comprise decreasing the frequency or dosage of the at least one immunosuppressant drug, or halting treatment with such a drug, or administering a milder immunosuppressant drug, for example. In some cases, following such adjustment of immunosuppressant therapy, the method is repeated to assess the effect of such therapy adjustment, for instance, after 1 week, 2 weeks, 1 month, 2 months, 3 months, 6 months or one year following the adjustment in the therapy. In some cases, if the method indicates that the recipient has AR or ADNR, a surveillance biopsy is ordered for the recipient, optionally, along with or prior to an adjustment in immunosuppressive therapy, such as increasing the frequency or dosage of the at least one immunosuppressant drug, administering a further immunosuppressant drug, or administering a different immunosuppressive drug to the recipient.

In some embodiments, the recipient may have undergone other biomarker testing, such as a liver function test, prior to conducting a method herein. For example, in the case of a liver transplant recipient, serum alanine aminotransferase concentration, serum aspartate aminotransferase concentration, alkaline phosphatase, direct bilirubin and/or total bilirubin levels may have been determined. For example, serum alanine aminotransferase concentration, serum aspartate aminotransferase concentration, alkaline phosphatase, direct bilirubin and/or total bilirubin levels, or changes in those parameters may be used to provide an indication of the performance of the liver transplant. The terms "level" and "concentration" herein are used interchangeably to refer to the concentration of particular proteins or other markers in a recipient's sample.

In some cases, a transplant recipient assessed in methods herein may have results from parameters such as those above indicating normal liver function, while in other cases, the recipient may have results indicating impairment in liver function or graft failure. For example, such tests in some cases may be performed just after transplantation in order to determine how the new liver is functioning, in order to establish a baseline for the functioning of the new liver. And such tests may, in some cases, be repeated in order to see whether the liver function changes over time. For example, parameters such as alanine aminotransferase concentration, aspartate aminotransferase concentration, alkaline phosphatase concentration, direct bilirubin concentration, or bilirubin concentration in serum may be determined. Certain levels of those parameters indicate normal liver function, with elevated levels indicating some form of impaired liver function. Impairment in liver function can also be indicated by an increase in those parameters compared to a post-transplant baseline level, such as at least a doubling of the parameter values, even if the values remain within the normal range. Alternatively, elevation of the parameter values to outside the normal range, may also indicate impaired liver function.

In some cases, a "baseline" against which an increase in alanine aminotransferase, alkaline phosphatase, or total bilirubin levels is measured is a time-point post transplantation at which serum alanine aminotransferase, alkaline phosphatase, or total bilirubin levels are at their lowest, often 1-2 weeks post-transplantation. For example, prior to liver transplantation, alanine aminotransferase may be very high, and then may fall to a nadir after transplantation once the transplanted liver functions. Changes in serum alanine aminotransferase, alkaline phosphatase, direct bilirubin and/or total bilirubin levels following this low baseline level may then be monitored to assess the continued function of the transplanted liver. In general, a doubling of one or more of these levels above baseline may be used to indicate a decline in liver function.

In some embodiments, the liver transplant recipient has a serum alanine aminotransferase level of less than or equal to 60 IU/L for a male recipient or less than or equal to 36 IU/L for a female recipient, or a serum alanine aminotransferase level indicative of non-rejection. In some embodiments, the liver transplant recipient has a serum alanine aminotransferase level of greater than 60 IU/L (male) or greater than 36 IU/L (female). In some such embodiments, the liver transplant recipient has a serum of alanine aminotransferase level of 10 to 40 IU/L. In some such embodiments, the liver transplant recipient has a serum alanine aminotransferase level of at most 30, at most 40, or at most 60 IU/L. In other embodiments, a male liver transplant recipient has a serum alanine aminotransferase level of at least 60, at least 80, at least 100, at least 120, at least 140, at least 160, at least 180, at least 200, at least 220, at least 240, at least 260, at least 280, or at least 300 IU/L. In some such embodiments, a male liver transplant recipient has a serum alanine aminotransferase level of at most 80, at most 100, at most 120, at most 140, at most 160, at most 180, at most 200, at most 220, at most 240, at most 260, at most 280, at most 300, or at most 350 IU/L. In some such embodiments, a male liver transplant recipient has a serum of alanine aminotransferase level of 60-350 IU/L, 60-200 IU/L, 100-350 IU/L, 100-200 IU/L, 80-350 IU/L, 80-200 IU/L, or 60-100 IU/L. In some embodiments, a female liver transplant recipient has a serum alanine aminotransferase level of at least 36, at least 40, at least 60, at least 80, at least 100, at least 120, at least 140, at least 160, at least 180, at least 200, at least 220, at least 240, at least 260, at least 280, or at least 300 IU/L. In some such embodiments, a female liver transplant recipient has a serum alanine aminotransferase level of at most 60, at most 80, at most 100, at most 120, at most 140, at most 160, at most 180, at most 200, at most 220, at most 240, at most 260, at most 280, at most 300, or at most 350 IU/L. In some such embodiments, a female liver transplant recipient has a serum of alanine aminotransferase level of 40-350 IU/L, 40-300 IU/L, 40-200 IU/L, 60-350 IU/L, 60-200 IU/L, 100-350 IU/L, 100-200 IU/L, 80-350 IU/L, 80-200 IU/L, or 60-100 IU/L. In some cases, the recipient's serum alanine aminotransferase level has at least doubled compared to a baseline level obtained following transplantation. A doubling of the serum alanine aminotransferase level may, in some embodiments, regardless of the baseline level, indicate loss of liver function due to rejection or other causes. In some cases, the increase in serum alanine aminotransferase (e.g., any increase in the concentration of serum alanine aminotransferase described herein) may occur over 1 day, 1 week, 2 weeks, 3 weeks, 1 month, 2 months, 3 months, 4 months, 5 months, or 6 months, 1 year, 1.5 years, 2 years, or more compared to the baseline level after transplantation.

In some embodiments, the liver transplant recipient has a serum alkaline phosphatase level of less than or equal to 200 IU/L, or a level that otherwise is in the normal range. In some embodiments, the liver transplant recipient has an alkaline phosphatase level of greater than 200 IU/L. In some such embodiments, the liver transplant recipient has an alkaline phosphatase level of at least 100, at least 110, at least 120, at least 130, at least 140, at least 150, at least 160, at least 170, at least 180, or at least 190 IU/L. In some such embodiments, the liver transplant recipient has an alkaline phosphatase level of no more than 120, no more than 150, no more than 160, no more than 170, no more than 180, no more than 190, or no more than 200 IU/L. In some such embodiments, the liver transplant recipient has an alkaline phosphatase level of 50 to 200 IU/L, 50-150 IU/L, 100-200 IU/L, or 50-100 IU/L. In other embodiments, the liver transplant recipient has a scrum alkaline phosphatase level of at least 200, at least 220, at least 240, at least 260, at least 280, at least 300, or at least 350 IU/L. In some such embodiments, the liver transplant recipient has an alkaline phosphatase level of no more than 220, no more than 240, no more than 260, no more than 280, no more than 300, or no more than 350 IU/L. In some such embodiments, the liver transplant recipient has an alkaline phosphatase level of 200-350 IU/L or 200-300 IU/L. In some cases, the recipient's alkaline phosphatase level has at least doubled compared to a baseline level obtained following transplantation. In other cases, the level has not doubled compared to such a baseline level. A doubling of the serum alkaline phosphatase level may, in some embodiments, regardless of the baseline level, may indicate loss of liver function due to rejection or other causes. In some cases, the increase in serum alkaline phosphatase may occur over 1 day, 1 week, 2 weeks, 3 weeks, 1 month, 2 months, 3 months, 4 months, 5 months, or 6 months, 1 year, 1.5 years, 2 years, or more compared to the baseline level after transplantation.

In some embodiments, the liver transplant recipient has a serum aspartate aminotransferase level of less than or equal to 40 IU/L, or otherwise a serum aspartate aminotransferase level indicating normal liver function. In some such embodiments, the liver transplant recipient has a serum aspartate aminotransferase level of at least 10, at least 20, at least 30, or less than 40 IU/L. In some embodiments, the liver transplant recipient has a serum aspartate aminotransferase level of 10-39 IU/L, 10-30 IU/L, 20-39 IU/L, or 5-39 IU/L. In some embodiments, the liver transplant recipient has a serum aspartate aminotransferase level of at most 20 IU/L, at most 30 IU/L, or at most 35 IU/L. In other embodiments, the liver transplant recipient has a serum of aspartate aminotransferase level of 40 IU/L or greater. In some such embodiments, the liver transplant recipient has a serum aspartate aminotransferase level of at least 40, at least 60, at least 80, at least 100, at least 120, at least 140, at least 160, at least 180, at least 200, at least 220, at least 240, at least 260, at least 280, or at least 300 IU/L. In some such embodiments, the liver transplant recipient has a serum aspartate aminotransferase level of at most 60, at most 80, at most 100, at most 120, at most 140, at most 160, at most 180, at most 200, at most 220, at most 240, at most 260, at most 280, or at most 300 IU/L. In some such embodiments, the liver transplant recipient has a serum aspartate aminotransferase level of 40-300 IU/L, 40-200 IU/L, 40-100 IU/L, 60-300 IU/L, 60-200 IU/L, or 60-150 IU/L. In some cases, the recipient's serum aspartate aminotransferase level has at least doubled compared to a baseline level obtained following transplantation. In other cases, the level has not doubled compared to such a baseline level. A doubling of the serum aspartate aminotransferase level may, in some embodiments, regardless of the baseline level, may indicate loss of liver function due to rejection or other causes. In some cases, the increase in serum aspartate aminotransferase level may occur over 1 day, 1 week, 2 weeks, 3 weeks, 1 month, 2 months, 3 months, 4 months, 5 months, or 6 months, 1 year, 1.5 years, 2 years, or more compared to the baseline level after transplantation.

In some instances, a transplant recipient may have total bilirubin concentration in serum of less than or equal to 1.5 mg/dL, or that otherwise indicates normal liver function. In some such cases, a recipient may have a total bilirubin concentration of, for example, less than 1.5 mg/dL, less than 1.4 mg/dL, less than 1.3 mg/dL, less than 1.2 mg/dL, less than 1.1 mg/dL, less than 1.0 mg/dL, less than 0.9 mg/dL, less than 0.8 mg/dL, less than 0.7 mg/dL, less than 0.6 mg/dL, or less than 0.5 mg/dL. In some instances, a transplant recipient may have direct bilirubin concentration in serum of less than 0.5 mg/dL, or that otherwise indicates normal liver function. In some cases, a recipient may have a direct bilirubin level of less than 0.4 mg/dL, less than 0.3 mg/dL, less than 0.2 mg/dL, or less than 0.1 mg/dL. In other cases, a recipient may have a total bilirubin level of greater than 1.5 mg/dL, such as greater than 1.7 mg/dL, greater than 1.8 mg/dL, or greater than 2.0 mg/dL. In some cases, a recipient may have a direct bilirubin level of 0.5 mg/dL or more, 0.6 mg/dL or more, 0.7 mg/dL or more, 0.8 mg/dL or more, 0.9 mg/dL or more, or 1.0 mg/dL or more. In some cases, regardless of the original baseline level, one or both of direct bilirubin and total bilirubin has at least doubled compared to the baseline level. In some cases, the increase in direct and/or total bilirubin level may occur over 1 day, 1 week, 2 weeks, 3 weeks, 1 month, 2 months, 3 months, 4 months, 5 months, or 6 months, 1 year, 1.5 years, 2 years, or more compared to the baseline level after transplantation.

In some instances, normal liver function tests (LFTs), such as alkaline phosphatase, aspartate aminotransferase, alanine aminotransferase, direct bilirubin and/or total bilirubin levels can be used to diagnose or determine likelihood of AR along with methods herein without depending on invasive biopsies or obtaining a sample of transplanted liver tissue. In some cases, the LFTs of the transplant recipient are stable (i.e., have not doubled) over at least 10 days, 20 days, 30 days, 40 days, 50 days, 60 days, 90 days, 100 days, 200 days, 300 days, -400 days or longer.

In some embodiments, the method is capable of distinguishing non-rejection from one or both of acute rejection (AR) and acute dysfunction without rejection (ADNR). In some embodiments, the method has an NPV of at least 70%, at least 71%, at least 72%, at least 73%, at least 74%, at least 75%, at least 76%, at least 77%, at least 78%, at least 79%, at least 80%, at least 81%, at least 82%,, at least 83%*,* at least 84%, at least 85%, at least 86%, at least 87%, at least 88%, at least 89%, at least 90%, at least 91%, at least 92%, at least 93%, at least 94%, at least 95%, at least 96%, or at least 97%. In some embodiments, the method further comprises detecting AR when the dd-cfNA comprises greater than or equal to 20.4% of total cfDNA. In some embodiments, ADNR comprises biliary obstruction, cholestasis, steatosis, drug-induced liver injury, or infection. In some embodiments, the method is capable of distinguishing between AR and no rejection with an NPV of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, or at least 97%. In some embodiments, the method further comprises detecting AR when the dd-cfDNA comprises greater than or equal to 15% of total cfDNA. In some embodiments, the recipient is indicated as having AR or ADNR by a level of dd-cfDNA that is greater than or equal to a pre-determined threshold of from 10% to 17%, from 13% to 17%, from 13% to 15%, from 15% to 17%, from 14% to 17%, from 14% to 16%, from 14%to 15%, from 15% to 16%, from 14.5% to 15.5%, or a pre-determined threshold of 10%, 11%, 12%, 13%, 14%, 14.5%, 15%, 15.5%, 16%, or 17%.

In some embodiments, the method is capable of distinguishing non-rejection from AR. In some embodiments, the method further comprises detecting AR when the dd-cfDNA comprises greater than or equal to 5.3% of total cfDNA. In some embodiments, the recipient is indicated as having a likelihood of non-rejection by a level of dd-cfDNA that is less than a pre-determined threshold of from 4% o to 7%, from 4% to 6%, from 5% to 7%, from 4.5% to 6.5%, from 4.5% to 5.5%, from 5% to 6%, from 5% to 5.5%, or less than a pre-determined threshold value of 4%, 4.5%, 5%, 5.1%, 5.2%, 5.3%, 5.4%, 5.5%, 6%, 6.5%, or 7%.

In some embodiments, the method is capable of distinguishing AR from ADNR. In some embodiments, the recipient is indicated as having a likelihood of AR by a level of dd-cfDNA that is greater than or equal to a pre-determined threshold of from 18% to 25%, from 18% to 23%, from 199% to 22%, from 19'/() to 21%, from 20% to 22%, from 20% to 21%, from 20.2% to 20.6%, or a pre-determined threshold of 18%, 19%, 20%, 20.2%, 20.4%, 20.6%, 20.8%, 21%, 22%, 23%, or 25%.

In some embodiments, the method further comprises detecting AR in the liver transplant recipient when the dd-cfDNA comprises greater than or equal to 5.3% of total cfDNA. In some embodiments, the method is capable of distinguishing between AR and non-AR, wherein the distinguishing has an NPV of at least 70%, at least 71%, at least 72%, at least 73%, at least 74%, at least 75%, at least 76%, at least 77%, at least 78%, at least 79%, at least 80%, at least 81%, at least 82%, at least 83%*,* at least 84%, at least 85%, at least 86%, at least 87%, at least 88%, at least 89%, at least 90%, at least 91%, at least 92%, at least 93%, at least 94%, at least 95%, at least 96%, or at least 97% or the distinguishing has a PPV of at least 50%, at least 55%, at least 58%, at least 60%, at least 62%, at least 64%, at least 66%, at least 68%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, or at least 97%.

In some embodiments, the method is capable of distinguishing between acute rejection (AR) and acute dysfunction without rejection (ADNR). In some embodiments, the method has an NPV of at least 70%, at least 71%, at least 72%, at least 73%, at least 74%, at least 75%, at least 76%, at least 77%, at least 78%, at least 79%, at least 80%, at least 81%, at least 82%, at least 83%*,* at least 84%, at least 85%, at least 86%, at least 87%, at least 88%, at least 89%, at least 90%, at least 91%, at least 92%, at least 93%, at least 94%, at least 95%, at least 96%, or at least 97%. In some embodiments, the method further comprises detecting AR when the dd-cfDNA comprises greater than or equal to 20.4% of total cfDNA. In some embodiments, ADNR comprises biliary obstruction, cholestasis, steatosis, drug-induced liver injury, or infection. In some embodiments, the method is capable of distinguishing between AR and TX with an NPV of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, or at least 97%. In some embodiments, the method further comprises detecting AR when the dd-cfDNA comprises greater than or equal to 15% of total cfDNA. In some embodiments, the method further comprises detecting non-TX when the dd-cfDNA comprises greater than or equal to 5.3% of total cfDNA.

In some embodiments, the method further comprises comprising repeating the method every 1-2 months. In some embodiments, the method further comprises suggesting one or more potential treatments for the recipient. In some embodiments, the method further comprises providing a report summarizing the recipient's level of dd-cfDNA. In some embodiments, the method further comprises selecting a treatment for the recipient based on the recipient's level of dd-cfDNA. In some embodiments, the method further comprises alteration in drug therapy based on the recipient's level of dd-cfDNA. In some embodiments, the method further comprises recommending administration of the new immunosuppressant drug when acute rejection is detected in the liver transplant recipient. In some embodiments, the method further comprises recommending ceasing administration of the new immunosuppressive drug when acute rejection is no longer detected in the liver transplant recipient. In some embodiments, the method further comprises detecting AR or ADNR in the liver transplant recipient when the dd-cfDNA comprises greater than or equal to 5.3% of total cfDNA. In some embodiments, the method further comprises detecting AR in the liver transplant recipient when the dd-cfDNA comprises greater than or equal to 15% of total cfDNA.

### Biomolecule Profiles

The methods herein may comprise specifically detecting, profiling, or quantitating biomolecules (e.g., nucleic acids, DNA,) that are within the biological samples to determine a gene or nucleic acid level profile. In some instances, genomic expression products, including RNA, or polypeptides, may be isolated from the biological samples. In some cases, nucleic acids, DNA, RNA, polypeptides may be isolated from a cell-free source. In some cases, nucleic acids, DNA, RNA, polypeptides may be isolated from cells derived from the transplant recipient. In some cases, the molecules detected are derived from molecules endogenously present in the sample via an enzymatic process (e.g. cDNA derived from reverse transcription of RNA from the biological sample followed by amplification).

In some embodiments, gene or nucleic acid levels can be determined using a probe array. A number of distinct array formats are available. Some arrays, such as an Affymetrix HG-U133 PM microarray or other Affymetrix GeneChip^{®} array, have different probes occupying discrete known areas of a contiguous support. Exemplary microarrays include but are not limited to the Affymetrix Human Genome U133 Plus 2.0 GeneChip or the HT HG-U133+ PM Array Plate.

In other methods, gene or nucleic acid levels can be determined by so-called "real time amplification" methods also known as quantitative PCR or Taqman. The basis for this method of monitoring the formation of amplification product formed during a PCR reaction with a template using oligonucleotide probes/oligos specific for a region of the template to be detected.

In some embodiments, nucleic acid from the sample is purified then amplified using PCR and appropriate primers. In some embodiments, whole genome application enrichment follows PCR amplification.

In some embodiments, gene or nucleic acid levels can be determined by sequencing, such as by DNA sequencing. Sequencing may be performed by any available method or technique. Sequencing methods may include: Next Generation sequencing (i.e., non-biased next generation sequencing), high-throughput sequencing, pyrosequencing, classic Sanger sequencing methods, sequencing-by-ligation, sequencing by synthesis, sequencing-by-hybridization, RNA-Seq (Illumina), Digital Gene Expression (Helicos), next generation sequencing, single molecule sequencing by synthesis (SMSS) (Helicos), Ion Torrent Sequencing Machine (Life Technologies/Thermo-Fisher), massively-parallel sequencing, clonal single molecule Array (Solexa), shotgun sequencing, single molecule nanopore sequencing, sequencing by ligation, sequencing by hybridization, sequencing by nanopore current restriction, Maxim-Gilbert sequencing, primer walking, or a combination thereof. Sequencing by synthesis may comprise reversible terminator sequencing, processive single molecule sequencing, sequential nucleotide flow sequencing, or a combination thereof. Sequential nucleotide flow sequencing may comprise pyrosequencing, pH-mediated sequencing, semiconductor sequencing or a combination thereof. Conducting one or more sequencing reactions may comprise whole genome sequencing or exome sequencing.

Sequencing reactions may comprise one or more capture probes or libraries of capture probes. At least one of the one or more capture probe libraries may comprise one or more capture probes to 1, 2, 3, 4, 5, 6 , 7, 8, 9, 10, 11, 12, 13, 14, 15, 20, 30, 40, 50, 60, 70, 80, 90, 100, 110, 120, 130, 140, 150, 160, 170, 180, 190, 200, 210, 220, 230, 240, 250 or more genomic regions. The libraries of capture probes may be at least partially complementary. The libraries of capture probes may be fully complementary. The libraries of capture probes may be at least 5%, 10%, 15%, 20%, %, 25%, 30%, 35%, 40%, 45%, 50%*,* 55%, 60%, 70%, 80%, 90%, 95%., 97% or more complementary.

Sequencing reactions may comprise one or more sets of amplification primers or libraries of amplification primers. At least one of the one or more amplification primer libraries may comprise one or more amplification primers to 1, 2, 3, 4, 5, 6 , 7, 8, 9, 10, 11, 12, 13, 14, 13, 20, 30, 40, 50, 60, 70, 80, 90, 100, 110, 120, 130, 140, 150, 160, 170, 180, 190, 200, 210, 220, 230, 240, 250 or more genomic regions. The libraries of amplification primers may be at least partially complementary. The libraries of amplification primers may be fully complementary. The libraries of amplification primers may be at least 5%, 10%, 15%, 20%, %, 25%, 30%, 35%, 40%, 45%, 50%, 55%, 60%, 70%, 80%, 90%, 95%, 97% or more complementary.

### dd-cfDNA Determination Methods

Methods herein involve determining dd-cfDNA in the sample, and, in particular, whether or not the level of dd-cfDNA, such as the percent dd-cfDNA out of total cfDNA in the sample, is at or above a particular pre-determined threshold indicating rejection. Accordingly, an "amount of dd-cfDNA" or "level of dd-cfDNA" may in some embodiments be reported as a percentage of the total cfDNA obtained from the sample.

There are several different methods for determining the amount of dd-cfNA such as dd-cfDNA in a sample. In some methods, the level of dd-cfDNA is determined by using genotyping data from both the donor and the recipient, for example, each obtained prior to the transplantation. In many other cases however, donor genotype data is not available. Thus, in some cases, only recipient genotype data is available and used in the method. For example, recipient genotyping may be performed on PBMC samples from the recipient. In yet other cases, neither the donor nor the recipient has been genotyped prior to determining the level of dd-cfDNA.

In some embodiments, dd-cfNA or dd-cfDNA level is determined by analysis of single nucleotide polymorphisms (SNPs) in the cfNA or cfDNA obtained from the sample. For example, a donor and a recipient may have certain different SNPs at particular genetic loci. In some embodiments multiple SNPs are evaluated, such as, for instance, at least 20, at least 50, at least 100, at least 200, at least 500, at least 1000, at least 2000, at least 5000, at least 10,000, at least 20,000, at least 50,000, at least 100,000, at least 200,000, at least 500,000, at least 1 million, at least 2 million, or at least 2.5 million SNPs. In some cases, the number of SNPs analyzed may vary from 20,000 to 100,000, 20,000 to 500,000, 20,000 to 50,000, 50,000 to 500,000, 50,000 to 100,000, 50,000 to 200,000, or 100,000 to 500,000. For example, in some embodiments, human genome chips or arrays may be utilized in methods herein, which may have up to 2.5 million analyzable SNPs. (See, e.g, Fig. 7.) In some cases, all or a subset of such SNPs may be evaluated. Thus, in some embodiments, sequencing of cfDNA is performed so as to evaluate the desired genomic loci or SNPs intended to be used in the analysis, which may or may not involve sequencing over the complete genome. And in some embodiments, the genotype information derived from the recipient (and, if available, from the donor) may be sufficient to cover the SNPs or genomic loci to be evaluated, and does not necessarily need to comprise the whole genome. In other cases, whole genome sequencing is performed on the cfDNA and/or to obtain the recipient's genotype information.

Where donor and/or recipient genotype data are available, i.e., a "two-genome" approach, particular SNP differences may be known prior to analysis. Alternatively, where genotype data for the donor and/or the recipient are not available, particular SNP differences may be found based on assaying for unique SNPs that occur in subjects with the same disease as the recipient, such as liver disease, with the expectation that the donor cfDNA will not show these unique SNPs. In some embodiments, a particular threshold is pre-determined based on clinical studies that compare predictions of rejection based on the specific dd-cfDNA analysis algorithm used to determine the percent dd-cfDNA to actual rejection based on a surveillance biopsy result.

Typically, the genome of the donor is unknown or unavailable, so a "two genomes" method cannot be performed. The methods described herein solve the problem of determining levels of dd-cfDNA while not knowing the genotype information of the donor. In an approach that does not rely on donor genotype information, to quantify the observed abundance of alleles of each genotyped SNP in cfDNA sequences by sequencing, low quality reads, reads that are not mapped uniquely to the genome, and reads with potential for mapping biased by genetic variability may be filtered. Duplicated reads are then removed and allele appearances of each genotyped SNP counted (e.g. by a SAMtools mpileup function). The observed allele appearances in cfDNA and the recipient genotype are the inputs for a "one-genome" model.

In such a one-genome model, to calculate the donor-derived cfDNA in one embodiment, the recipient genotype is first calculated. Recipient genotype can depend on the recipient measured genotype and the genotyping error rate. Since vital organ transplants are rarely closely related, the model can assume that the donor genotype is randomly selected from one of the human populations and super-populations, for example, from an available database such as the 1000 Genomes Project (available from the International Genome Sample Resource (IGSR)). Given this assumption, the probability of a specific donor allele is its frequency in the selected population (Hardy-Weinberg equilibrium). The algorithm, in some embodiments, then iterates over the 1000 Genomes Project populations and super-populations to detect the most likely ancestral population of the donor. For example, the 1000 Genomes Project segregates human genomes in its model into 32 general subtypes. The probability of an allele type observed at any genotyped location depends on the true genotype of the recipient, the donor population type and the fraction of the donor-derived cfDNA. The algorithm iterates over all the 32 population types and selects the one which maximizes the likelihood of the observed data. Finally, the log-likelihood of the data can be computed by summing log-likelihoods over all SNPs, assuming SNPs are independent (this assumption is also made by the two-genomes method). An optimization algorithm is then used to find the maximum likelihood parameter values and the donor-fraction of the total cfDNA.

In some instances, this procedure can be executed in a parallelized fashion, dramatically speeding up the determination of dd-cfDNA in multiple samples or sequencing reactions (e.g. from the same individual or from multiple individuals).

An example of a "one-genome" dd-ofDNA determination without donor genotype information is as shown in Figures 6 and 7, and an associated algorithm for determining the level of dd-cfDINA is described, for example, in US Patent Application Publication No. US2021/0115506 A1 and WO2018187226A1, which are each incorporated in their entirety by reference herein.

Accordingly, in some embodiments, the level of dd-cfNA, such as dd-cfDNA is determined by sequencing the of DNA, and inputting into a computer-implemented algorithm (i) cfDNA sequence information at a plurality of genetic loci, and (ii) genotype information from the recipient, in the absence of genotype information from the donor, wherein the algorithm identifies dd-cfDNA sequences at the plurality of genetic loci by identifying a pattern of cfDNA sequences at the loci that differ from the cfDNA sequences of the recipient but that correlate with a pattern of human DNA sequences found in a database of human genomes, i.e. such as from the 1000 Genomes Project populations. (See Fig. 7.)

In some embodiments herein, methods comprise determining the level of donor derived cell-free DNA (dd-cfDNA) in the cfDNA in the recipient's sample, and distinguishing one or both of AR and ADNR from non-rejection in the recipient based upon results from an algorithm that considers the level of dd-cfDNA and provides a result indicating rejection or non-rejection. Thus, for example, a trained algorithm may be used that accounts for the dd-cfDNA level. In some embodiments, the trained algorithm may be used that accounts for the dd-cfDNA level and other measurements from LFTs. In some embodiments, the trained algorithm may be used that accounts for the dd-cfDNA level and serum alanine aminotransferase concentration, serum aspartate aminotransferase concentration, serum alkaline phosphatase level, and/or total bilirubin concentration. In some embodiments, the trained algorithm may be used that accounts for the dd-cfDNA level and serum alanine aminotransferase concentration. In some embodiments, the trained algorithm may be used that accounts for the dd-cfDNA level and serum alanine aminotransferase concentration.

### Trained Algorithms

In some embodiments, methods include using a trained algorithm to analyze sample data, particularly to detect or rule-out AR and/or ADNR. In some embodiments, methods comprise applying a trained algorithm to the level dd-cfDNA and determining a result of the algorithm, wherein the result indicates rejection or non-rejection. In some embodiments, the level of dd-cfDNA is determined using a trained algorithm. A "trained algorithm" or "training algorithm," as used herein, is an algorithm that is developed based on a set of training data, such as dd-cfDNA levels samples from subjects, with AR, with ADNR, or without rejection, and SNP information for SNPs throughout a genome that may differ between a donor and recipient, and developed to use the data to distinguish data profiles associated with different outcomes or phenotypes, such as rejection, ADNR, and non-rejection.

In such supervised learning approaches, a group of samples from two or more groups (e.g. rejection and non-rejection, as well as types of rejection such as acute cellular rejection and antibody mediated rejection) are analyzed with a statistical classification method. Differential gene or nucleic acid level data can be discovered that can be used to build a classifier that differentiates between the two or more groups, such as rejection and non-rejection. A new sample can then be analyzed so that the classifier can associate the new sample with one of the two or more groups. Examples of trained algorithms include without limitation a neural network (multi-layer perceptron), support vector machine, k-nearest neighbors, Gaussian mixture model, Gaussian, naive Bayes, decision tree and radial basis function (RBF). Linear classification methods include Fisher's linear discriminant, LDA, logistic regression, naive Bayes classifier, perceptron, and support vector machines (SVMs). Other algorithm methods compatible with the invention include quadratic classifiers, k-nearest neighbor, boosting, decision trees, random forests, neural networks, pattern recognition, Elastic Net, Golub Classifier, Parzen-window, Iterative RELIEF, Classification Tree, Maximum Likelihood Classifier, Nearest Centroid, Prediction Analysis of Microarrays (PAM), Fuzzy C-Means Clustering, Bayesian networks and Hidden Markov models.

Classification by a trained algorithm using supervised methods is performed in some embodiments by the following methodology:

In order to solve a given problem of supervised learning, one can consider various steps:
1. Gather a training set. These can include, for example, samples that are from recipients with known rejection, known ADNR, and with known non-rejection, and in some cases also normal subjects, and/or subjects with particular types of rejection such as acute cellular rejection and antibody mediated rejection. These training samples are used to "train" the classifier.
2. Determine the input "feature" representation of the learned function. The accuracy of the learned function depends on how the input object is represented. Typically, the input object is transformed into a feature vector, which contains a number of features that are descriptive of the object. The number of features should not be too large, because of the curse of dimensionality; but should be large enough to accurately predict the output.
3. Determine the structure of the learned function and corresponding learning algorithm. A learning algorithm is chosen, e.g., artificial neural networks, decision trees, Bayes classifiers or support vector machines. The learning algorithm is used to build the classifier.
4. Build the classifier (e.g. classification model). The learning algorithm is run on the gathered training set. Parameters of the learning algorithm may be adjusted by optimizing perfonnance on a subset (called a validation set) of the training set, or via cross-validation. After parameter adjustment and learning, the performance of the algorithm may be measured on a test set of naive samples that is separate from the training set.

Once the classifier (e.g. classification model) is determined as described above, it can be used to classify a sample, e.g., that of a liver transplant recipient analyzed by the methods of the invention from such a recipient.

Training of multi-dimensional algorithms may be performed using numerous samples. For example, training may be performed using at least 10, 20, 30, 40, 50, 60, 70, 80, 90, 100, 110, 120, 130, 140, 150, 160, 170, 180, 190, 200 or more samples from subjects with known rejection or non-rejection outcomes. In some cases, training of the multi-dimensional algorithms may be performed using at least 200, 210, 220, 230, 240, 250, 260, 270, 280, 290, 300, 350, 400, 450, 500 or more samples. In some cases, training may be performed using at least 525, 550, 600, 650, 700, 750, 800, 850, 900, 950, 1000, 1100, 1200, 1300, 1400, 1500, 1600, 1700, 1800, 2000 or more samples.

As trained algorithms require manipulation of many parameters simultaneously, often tens or hundreds of parameters, tracking SNPs, for example, they are developed and calculated using appropriate software programming methods, and may be implemented on a computer. A further discussion of computer and software implements that may be used to compute or develop a trained algorithm is provided further below.

### Methods of treating liver transplant recipients

In some instances, the methods described herein provide information to a medical practitioner that can be useful in making a therapeutic decision. Therapeutic decisions may include decisions to: continue with a particular therapy, modify a particular therapy, alter the dosage of a particular therapy, stop or terminate a particular therapy, altering the frequency of a therapy, introduce a new therapy, introduce a new therapy to be used in combination with a current therapy, or any combination of the above. Furthermore, the methods used in this disclosure may guide the decision points in treatment regimens (e.g. addition of agents to the immunosuppression regimen due to increased evaluation of risk). For example, they may allow the evaluation of a patient with low time-of-transplant risk factors (e.g. high HLA matching between recipient and donor organ), or as having AR, or non-rejection, justifying the adjustment of a treatment regimen.

In particular embodiments, methods herein may be used to determine whether a recipient of a liver transplant should or should not receive a surveillance biopsy. For example, in some embodiments, a recipient with a non-rejection result according to the methods herein, such as a negative result in the dd-cfDNA analyses, may be determined to not be in need of a biopsy, whereas a recipient who is positive in one or both analyses may be determined to need a biopsy.

Thus, methods herein also include methods of treating a liver transplant recipient, wherein the recipient is determined to have a likelihood of rejection according to a method of distinguishing non-rejection from one or both of AR and ADNR herein. In some embodiments, the method results indicate that the recipient is likely to have AR. In other embodiments, the method results indicate that the recipient is likely to have either AR or ADNR. In other cases, the method results indicate that the recipient is likely to have ADNR. In some embodiments, the recipient is receiving at least one immunosuppressive drug. In some embodiments, the treatment method comprises increasing the frequency or dosage of the at least one immunosuppressant drug, administering a further immunosuppressant drug, or administering a different immunosuppressive drug to the recipient if the recipient has a positive result (i.e., indicating AR or ADNR). In some cases, if the recipient's test results indicate by dd-cfDNA and optionally also by LFT analyses indicate AR or ADNR, the method of treatment comprises performing a surveillance biopsy. In some cases, the dd-efDNA analysis is performed prior to a surveillance biopsy and such a biopsy is not ordered for the recipient unless the test, optionally in combination with an LFT analysis, provides a positive result. In some cases, the recipient does not show clinical signs of rejection at the time that the recipient's sample is obtained for the dd-cfDNA analysis to be performed.

Many different drugs are available for treating liver transplant rejection, such as immunosuppressive drugs used to treat transplant rejection, such as calcineurin inhibitors (e.g., cyclosporine, tacrolimus), mTOR inhibitors (e.g., sirolimus and everolimus ), anti-proliferatives (e.g., azathioprine, mycophenolic acid, mycophenolate mofetil or MMF), corticosteroids (e.g., prednisone, prednisolone , and hydrocortisone), antibodies (e.g., rituximab, basiliximab, daclizumab, muromonab-CD3, alemtuzumab, anti-thymocyte globulin and anti-lymphocyte globulin), intravenous or subcutaneous immunoglobulins (IVIG), rabbit antithymocyte globulin (rATG), interleukin 2 (IL2) receptor antagonists (e.g. basiliximab) or daclizumab), and biologics (e.g. belatacept), or combinations of one or more of the above. In some embodiments, a recipient may be receiving a standard of care treatment post-transplant. An additional immunosuppressant regimen to note is a "breakout" regimen used for treatment of any rejection episodes that occur after organ transplant. This may be a permanent adjustment to the maintenance regimen or temporary drug therapy used to minimize damage during the acute rejection episode. The adjustment may comprise temporary or long-term addition of a corticosteroid, temporary or long-term use of an immunosuppressive drug, temporary use of lymphocyte-depleting agents, and long-term addition of antiproliferative agents (e.g. mycophenolate mofetil/MMF or azathioprine, for patients not already receiving it), or increase in the maintenance dose of CNI or mTOR inhibitor. Breakout treatment may also comprise plasma exchange, intravenous immunoglobulin, and anti-CD-20 antibody therapy, and any combination thereof.

With respect to immunosuppression therapy of liver transplant recipients, the Charlton et al. (Transplantation, 2018 May;102(5):727-743) outline example immunosuppression regimens for liver transplant recipients. Following transplant, a patient may receive an "induction" regimen comprising at least intravenous corticosteroids for several days until oral corticosteroids can be initiated; this regimen can comprise a biologic agent such as an IL-2 receptor antagonist (e.g. basiliximab or daclizumab) or a lymphocyte-depleting agent (e.g. antithymocyte globulin, antilymphocyte globulin, alemtuzumab, and/or monomurab-CD3) in the case of individuals with kidney dysfunction or higher immunological risk (e.g. retransplantation for rejection, immune-mediated liver disease, simultaneous liver-kidney transplant, or those highly sensitized). After transplant, a patient may be treated with a maintenance immunosuppression regimen which can comprise a calcineurin inhibitor (e.g. tacrolimus or cyclosporine) or an mTOR inhibitor (e.g. sirolimus) and an antiproliferative agent (e.g. mycophenolic acid or azathioprine). The initial maintenance regimen may optionally additionally comprise a corticosteroid. Given an initial period after transplantation with no acute rejection, the immunosuppression regimen may be adjusted to a long-term maintenance phase, where the lowest planned doses of immunosuppressants can be used.

In some embodiments, methods comprise treating a liver transplant recipient, optionally wherein the recipient has received at least one steroid or immunosuppressant drug, wherein the recipient has previously been determined to have acute rejection (AR) or acute disfunction non-rejection (ADNR) according to a process herein, such as comprising: (a) obtaining a sample from a liver transplant recipient; (b) obtaining cfNA or cfDNA from the sample; (c) determining a level of dd-cfNA or dd-cfDNA in the cfNA or of DNA; and (d) distinguishing non-rejection from one or both of AR and ADNR in the recipient by comparing the level of dd-cfNA or dd-cfDNA to a pre-determined threshold value; wherein the method comprises administering at least one immunosuppressant drug. In other cases, the method comprises both administering at least one immunosuppressant drug to the recipient as well as conducting the process of steps (a)-(d) above prior to drug administration, after drug administration, or both. For example, methods herein may include treating a liver transplant recipient, optionally wherein the recipient has received at least one steroid or immunosuppressant drug, the method comprising (I) determining that the recipient has acute rejection (AR) or acute disfunction non-rejection (ADNR) by a process comprising: (a) obtaining a sample from a liver transplant recipient; (b) obtaining cfNA or cfDNA from the sample; (c) determining a level of dd-cfNA or dd-cfDNA in the cfNA or cfDNA; and (d) distinguishing non-rejection from one or both of AR and ADNR in the recipient by comparing the level of dd-cfNA or dd-cfDNA to a pre-determined threshold value, and (II) administering at least one immunosuppressant drug to the recipient. In some embodiments, the immunosuppressant drug comprises tacrolimus, an mTOR inhibitor, cyclosporine, a calcineurin inhibitor, methylprednisone, prednisone, mycophenolate, mycophenolic acid, intravenous immunoglobulin, rituximab, bortezomib, eculizumab, azathioprine, or an anti-B-cell antibody.

In some embodiments, a method of treatment herein, if the results indicate absence of rejection, methods herein may comprise either ordering the re-performance of the processes of (a)-(d) above or directly re-performing those processes at regular intervals, such as 1 week, 2 weeks, 3 weeks, 4 weeks, 2 months, 3 months, 4 months, 5 months, or 6 months, as part of a plan of active surveillance. "Active surveillance" herein refers to a treatment plan comprising regular physician visits, and optionally, regular diagnostic testing, to monitor a recipient for signs of rejection and/or organ dysfunction over a period of time. In some cases, the subject may be receiving immunosuppressive therapy, while in other cases the recipient may not be receiving therapeutics. For example, if rejection is not detected according to the methods herein, suitable active surveillance methods of treatment may include retraining from biopsy procedures or immunosuppressant regimen adjustments for a specific period of time, such as e.g. 1 week, 2 weeks, 3 weeks, 4 weeks, 2 months, 3 months, 4 months, 5 months, or 6 months. In some cases, where a recipient is receiving immunosuppressive therapy and the methods herein indicate non-rejection, the current immunosuppressive therapy may be maintained, or may be reduced, such as through administration of a lower dose of the current drugs or by an alteration in the drugs being administered. In some cases, when rejection is not detected and the patient has previously received an increase in dose of a particular immunosuppressant of their regimen, the current increase in dose or new immunosuppressant administration may be maintained or reduced.

If a patient is indicated as having a likelihood of AR, the physician can subject the patient to additional testing including performing a liver biopsy or performing other analyses such as ALT or AlkPrate at increased frequency. Additionally or alternatively, the physician can change the treatment regime being administered to the patient. A change in treatment regime can include administering an additional or different drug to a patient, or administering a higher dosage or frequency of a drug already being administered to the patient. Alternatively, if a patient is indicated as having or being at decreased risk of AR, the physician can change the treatment regime to administer a lower dosage or frequency of a drug already being administered to the patient.

In some embodiments, the present disclosure provides for a method treating a liver transplant recipient, optionally wherein the recipient has received at least one steroid or immunosuppressant drug, wherein the recipient has previously been determined to have acute rejection (AR) or acute disfunction non-rejection (ADNR) according to a process comprising: (a) obtaining a sample from a liver transplant recipient; (b) obtaining cfNA or cfDNA from the sample; (c) determining a level of dd-cfNA or dd-cfDNA in the cfNA or cfDNA; (d) distinguishing non-rejection from one or both of AR and ADNR in the recipient by comparing the level of dd-cfNA or dd-cfDNA to a pre-determined threshold value; wherein the method comprises administering at least one immunosuppressant drug. In some embodiments, the present disclosure provides a method of treating a liver transplant recipient, optionally wherein the recipient has received at least one steroid or immunosuppressant drug, the method comprising (I) determining that the recipient has acute rejection (AR) or acute disfunction non-rejection (ADNR) by a process comprising: (a) obtaining a sample from a liver transplant recipient; (b) obtaining cfNA or cfDNA from the sample; (c) determining a level of dd-cfNA or dd-cfDNA in the cfNA or cfDNA; (d) distinguishing non-rejection from one or both of AR and ADNR in the recipient by comparing the level of dd-cfNA or dd-cfDNA to a pre-determined threshold value, and (II) administering at least one immunosuppressant drug to the recipient. In some such embodiments, the immunosuppressant drug may comprise one or more calcineurin inhibitors (e.g., cyclosporine, tacrolimus), mTOR inhibitors (e.g., sirolimus and everolimus ), anti-proliferatives (e.g., mycophenolate, mycophenolic acid, mycophenolate mofetil or MMF), corticosteroids (e.g., prednisone, prednisolone, and hydrocortisone), and antibodies (e.g., rituximab, eculizumab, intravenous immunoglobulin, anti-thymocyte globulin and anti-lymphocyte globulin).

In some cases, methods herein may be repeated regularly as part of monitoring a recipient. Such monitoring may occur at different intervals, for example the monitoring may be daily, weekly, monthly, yearly, or some other time period, such as twice a month, three times a month, every two months, every three months, every 4 months, every 5 months, every 6 months, every 7 months, every 8 months, every 9 months, every 10 months, every 11 months, or every 12 months. For example, if methods herein are conducted on a regular basis, they can provide an indication whether an existing immunosuppressive regimen is working, whether the immunosuppressive regimen should be changed (e.g. via administration of a new immunosuppressant to the transplant recipient or increase in dose of an immunosuppressant currently being administered to the transplant recipient) or whether a biopsy or increased monitoring by other rejection markers such as creatinine or glomerular filtration rate should be performed. In some cases, consecutive (e.g. at least two) tests positive for rejection as described herein indicate that an additional action be taken, e.g. adjustment of the immunosuppressive regimen (e.g. via administration of a new immunosuppressant to the transplant recipient or increase in dose of an immunosuppressant currently being administered to the transplant recipient), collection and evaluation of a biopsy, further biomarker testing such as analysis of LFTs. In some cases, consecutive (e.g. at least two, three, four, five, six, seven, eight, nine, ten) tests ambiguous for rejection vs. non-rejection as described herein may indicate that an additional confirmatory action be taken, e.g. collection and evaluation of a biopsy or further biomarker testing such as an LFT. The consecutive (e.g. at least two, three, four, five, six, seven, eight, nine, ten) tests may be separated by an appropriate time period (e.g. one day, one week, two weeks, three weeks, one month, two months, three months, four months, five months, six months, or one year) to ensure that the tests accurately represent a trend.

Methods provided herein also encompass conducting a dd-cfDNA analysis on a transplant recipient who has already undergone a surveillance biopsy of the liver and received a biopsy result in the form of a histological analysis or a molecular profiling analysis. In some particular instances, the analysis of the biopsy (e.g., by histology or molecular profiling) may result in ambiguous, inconclusive or borderline results. In such cases, a method provided herein may assist a caregiver with determining whether the transplant recipient has acute rejection or with interpreting the biopsy. In other cases, the biopsy itself may be inconclusive or ambiguous, and in such cases the molecular analysis of the biopsy may be used in adjunct with the histology to confirm a diagnosis. In some instances, the analysis of the biopsy may yield a negative result. In such cases, the subject may receive a dd-cfDNA analysis as provided herein in order to confirm the negative result, or to distinguish non-rejection from one or both of AR and ADNR. In some cases, after receiving any type of biopsy result (e.g., negative result, ambiguous, inconclusive, borderline, positive), the patient may receive multiple, serial dd-cfDNA analyses as described herein, optionally in combination with other tests such as LFT analysis, in order to monitor changes in molecular markers correlated with acute rejection.

Treatment methods provided herein also include performing a biopsy on a transplant recipient who has received a dd-cfDNA analysis as described herein. In some embodiments, the recipient's dd-cfDNA results are positive, indicating AR or ADNR. In some cases, the recipient's dd-cfDNA results indicate AR. In some cases, a dd-cfDNA analysis provides a borderline result (i.e., at or near the threshold separating rejection from AR or ADNR). In such cases, the patient's healthcare worker may use the results of a biopsy test as a complement in order to confirm whether rejection is present.

### Computer implemented methods and systems for conducting methods herein

As described previously, gene or nucleic acid levels can be analyzed and associated with status of a subject (e.g., presence or absence of rejection) in a digital computer, while algorithms herein, such as trained algorithms may be applied through use of a computer. As shown in **Figure 5**, in some embodiments, a sample is first collected from a subject (for example, from a liver transplant recipient). The sample is assayed and nucleic acid products are generated. A computer system is used in analyzing the data and making a classification of rejection or non-rejection based on, for example, results from the dd-cfDNA level, wherein rejection is indicated by result of an algorithm accounting for the dd-cfDNA level data..

In some embodiments of the disclosure, a system that is capable of determining the level of dd-cfDNA is used to conduct methods herein. In some cases, such a system may include components for conducting assays to determing the level of dd-cfDNA. In some cases, alternatively or additionally, a system may include a computer and appropriate software for conducting one or more algorithms, such as trained algorithms, in order to determine the level of dd-cfDNA from a recipient sample. A system may comprise software that provides an algorithm result for a recipient, for example, positive or negative (i.e. rejection or no rejection), for the dd-cfDNA analyses, which may then in some embodiments be provided to a caregiver for the recipient in order to determine further treatment steps for the recipient.

Optionally, in a system herein, a computer is directly linked to a scanner or the like receiving experimentally determined signals related to gene or nucleic acid levels (i.e., for SNP identification), and the like. Alternatively, gene or nucleic acid levels can be input by other means. The computer can be programmed to convert raw signals into gene or nucleic acid levels (absolute or relative), compare measured gene or nucleic acid levels with one or more reference levels, or a scale of such values, as described above. The computer can also be programmed to assign values or other designations to gene or nucleic acid levels based on the comparison with one or more reference gene or nucleic acid levels, and to aggregate such values or designations for multiple gene or nucleic acids in a profile. The computer can also be programmed to output a value or other designation providing an indication of presence rejection or non-rejection as well as any of the raw or intermediate data used in determining such a value or designation.

The methods or systems provided herein may also be capable of generating and transmitting results through a computer network. As shovvn in **Figure 5****,** a sample is first collected from a subject (e.g. transplant recipient). The sample is assayed and gene or nucleic acid levels are generated. A computer system is used in analyzing the data and making classification of the sample. The result is capable of being transmitted to different types of end users via a computer network. In some instances, the subject (e.g. patient) may be able to access the result by using a standalone software and/or a web-based application on a local computer capable of accessing the internet. In some instances, the result can be accessed via a mobile application provided to a mobile digital processing device (e.g. mobile phone, tablet, etc.). In some instances, the result may be accessed by physicians and help them identify and track conditions of their patients. In some instances, the result may be used for other purposes such as education and research.

The methods or systems disclosed herein may include at least one computer program, or use of the same. A computer program may include a sequence of instructions, executable in the digital processing device's CPU, written to perform a specified task. Computer readable instructions may be implemented as program modules, such as functions, objects, Application Programming Interfaces (APIs), data structures, and the like, that perform particular tasks or implement particular abstract data types. In light of the disclosure provided herein, those of skill in the art will recognize that a computer program may be written in various versions of various languages.

The functionality of the computer readable instructions may be combined or distributed as desired in various environments. The computer program will normally provide a sequence of instructions from one location or a plurality of locations. In various embodiments, a computer program includes, in part or in whole, one or more web applications, one or more mobile applications, one or more standalone applications, one or more web browser plug-ins, extensions, add-ins, or add-ons, or combinations thereof.

Further disclosed herein are systems for classifying one or more samples and uses thereof. The system may comprise (a) a digital processing device comprising an operating system configured to perform executable instructions and a memory device; (b) a computer program including instructions executable by the digital processing device to classify a sample from a subject comprising: (i) a first software module configured to receive a an gene or nucleic acid level profile of one or more genes from the sample from the subject (; (ii) a second software module configured to analyze the gene or nucleic acid level profile from the subject; and (iii) a third software module configured to classify the sample from the subject based on a classification system comprising two or more classes (e.g. rejection vs. non-rejection).

A computer system herein may be in communication with a processing system. The processing system can be configured to implement the methods disclosed herein. In some examples, the processing system is a microarray scanner. In some examples, the processing system is a real-time PCR machine (optionally microfluidic). In some examples, the processing system is a nucleic acid sequencing system, such as, for example, a next generation sequencing system (e.g., Illumina sequencer, Ion Torrent sequencer, Pacific Biosciences sequencer, BGI sequencing system). The processing system can be in communication with the system through the network, or by direct (e.g., wired, wireless) connection. The processing system can be configured for analysis, such as nucleic acid sequence analysis.

Methods as described herein can be implemented by way of machine (or computer processor) executable code (or software) stored on an electronic storage location of the computer system, such as, for example, on the memory or electronic storage unit. During use, the code can be executed by the processor. In some examples, the code can be retrieved from the storage unit and stored on the memory for ready access by the processor. In some situations, the electronic storage unit can be precluded, and machine-executable instructions are stored on memory.

Systems herein for conducting the methods may include a digital processing device, or use of the same. In further embodiments, the digital processing device includes one or more hardware central processing units (CPU) that carry out the device's functions. In still further embodiments, the digital processing device further comprises an operating system configured to perform executable instructions. In some embodiments, the digital processing device is optionally connected a computer network. In further embodiments, the digital processing device is optionally connected to the Internet such that it accesses the World Wide Web. In still further embodiments, the digital processing device is optionally connected to a cloud computing infrastructure. In other embodiments, the digital processing device is optionally connected to an intranet. In other embodiments, the digital processing device is optionally connected to a data storage device.

In accordance with the description herein, suitable digital processing devices include, by way of non-limiting examples, server computers, desktop computers, laptop computers, notebook computers, sub-notebook computers, netbook computers, netpad computers, set-top computers, handheld computers, Internet appliances, mobile smartphones, tablet computers, personal digital assistants, video game consoles, and vehicles.

The digital processing device will normally include an operating system configured to perform executable instructions. The operating system is, for example, software, including programs and data, which manages the device's hardware and provides services for execution of applications. Exemplary operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}as well as personal computer operating systems such as Microsoft^{®} Windows^{®} Apple^{®} Mac OS X^{®}, UNIX^{®}, and UNIX-like operating systems such as GNU/Linux^{®}. In some embodiments, the operating system is provided by cloud computing. Mobile smart phone operating systems include, by way of non-limiting examples, Nokia^{®} Symbian^{®} OS, Apple^{®} iOS^{®}, Research In Motion^{®} BlackBerry OS^{®}, Google^{®} Android^{®}, Microsoft^{®} Windows Phone^{®} OS, Microsoft^{®} Windows Mobile^{®} OS, Linux^{®}, and Palm^{®} WebOS^{®}.

The digital processing device generally includes a storage and/or memory device. The storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the digital processing device is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, or other external memory devices.

A digital processing device may also include a display to send visual information to a user.

The digital processing device may include an input device to receive information from a user, e.g. from a keyboard or a touch screen, or other means of inputing information.

### Computer programs

The methods, kits, and systems disclosed herein may include one or more non-transitory computer readable storage media encoded with a program including instructions executable by the operating system to perform and analyze the test described herein; preferably connected to a networked digital processing device. A non-transitory computerreadable storage media may be encoded with a computer program including instructions executable by a processor to create or use an algorithm to determine one or more results for methods herein (i.e. levels of dd-cfDNA or parameters needed to determine level of dd-cfDNA). The storage media may comprise a database, in a computer memory, of one or more clinical features of control samples, for example, or of other data or parameters used in algorithms of the methods, or in creating a trained algorithm.
In some embodiments, a computer program includes a web application. In light of the disclosure provided herein, those of skill in the art will recognize that a web application, in various embodiments, utilizes one or more software frameworks such as Microsoft^{®} .NET or Ruby on Rails (RoR), and one or more database systems including, by way of non-limiting examples, relational, non-relational, object oriented, associative, and XML database systems. In further embodiments, suitable relational database systems include, by way of non-limiting examples, Microsoft^{®} SQL Server, mySQL^{™}, and Oracle^{®}.

In some embodiments, a computer program includes a mobile application provided to a mobile digital processing device. In some embodiments, the mobile application is provided to a mobile digital processing device at the time it is manufactured. In other embodiments, the mobile application is provided to a mobile digital processing device via the computer network described herein.

In some embodiments, a computer program includes a standalone application, which is a program that is run as an independent computer process, not an add-on to an existing process, e.g., not a plug-in. Those of skill in the art will recognize that standalone applications are often compiled. A compiler is a computer program(s) that transforms source code written in a programming language into binary object code such as assembly language or machine code. Suitable compiled programming languages include, by way of non-limiting examples, C, C++, Objective-C, COBOL, Delphi, Eiffel, Java^{™}, Lisp, Python^{™}, Visual Basic, and VB .NET, or combinations thereof. Compilation is often performed, at least in part, to create an executable program. In some embodiments, a computer program includes one or more executable complied applications.

In some embodiments, the computer program includes a web browser plug-in. In computing, a plug-in is one or more software components that add specific functionality to a larger software application. Examples of web browser plug-ins include Adobe^{®} Flash^{®} Player, Microsoft^{®} Silverlight^{®}, and Apple^{®} QuickTime^{®}.

Software modules connected with method herein may be created by techniques known to those of skill in the art using machines, software, and languages known to the art.

### Databases

The methods, kits, and systems disclosed herein may comprise one or more databases, or use of the same. Exemplary databases include, by way of non-limiting examples, relational databases, non-relational databases, object oriented databases, object databases, entity-relationship model databases, associative databases, and XML databases. In some embodiments, a database is internet-based. In further embodiments, a database is web-based. In still further embodiments, a database is cloud computing-based. In other embodiments, a database is based on one or more local computer storage devices.

### Data transmission and reports

Methods herein may further comprise providing one or more reports, and systems herein for conducting such methods may include means for generating such reports. The one or more reports may comprise a status or outcome of a transplant in a subject, i.e. whether the method indicates rejection or non-rejection. The one or more reports may also comprise information pertaining to therapeutic regimens for use in treating transplant rejection or in suppressing an immune response in a recipient, such as based on the results of the method. The one or more reports may be transmitted to a recipient or to a medical representative of the recipient such as a physician, physician's assistant, nurse, or other medical personnel, or to a family member, guardian, or legal representative of the subject.

### EXAMPLES

### Example 1. - Donor-Derived Cell-Free DNA Distinguishes Acute Rejection From Other Causes Of Graft Injury In Liver Transplant Recipients

Compared to other organ transplants, donor-derived cell-free DNA (dd-cfDNA) has not been tested robustly as an acute rejection (AR) biomarker in liver transplant recipients (LTR). The aim of this study was to evaluate the ability of dd-cfDNA in distinguishing AR from other causes (ADNR - acute dysfunction no rejection) before and at the time of graft dysfunction. Plasma tubes collected and stored from two LT biorepository cohorts (Northwestern University (NU); multicenter NIAID CTOT-14) were analyzed for dd-cfDNA quantification. Patient phenotypes included AR (N=57), ADNR (N=68), control LT recipients with normal graft function (TX; N=94), as well as non-AR (combination of ADNR+TX; N=162). In the training set (70% of cohort), dd-cfDNA levels were significantly different between AR vs. TX (AUC 0.95, 5.3% cutoff), AR vs. non-AR (AUC 0.85, 11.9% cutoff) and AR vs. ADNR (AUC 0.71, 20.4% cutoff). With the same cutoffs, in the test set (30% of cohort), the accuracy of AR vs. non-AR was 78.9% with NPV of 94.8% and AR vs. ADNR 63.8% accuracy with negative predictive value (NPV) of 87.8%. CTOT-14 subjects had serial collections prior to all diagnostic phenotypes. Analysis of dd-cfDNA in a subset of samples (using the cutoff lowest cutoff of 5.42%) demonstrated 62.5% sensitivity for AR (N=7) combined with ANDR (N=9); sensitivity for TX (N=40) was 70%. For discordant samples, the change (as a percentage of dd-cfDNA) was positive for all AR and ADNR phenotypes and negative for 10 of 12 TX phenotypes.

In the current study, plasma samples prospectively collected from two wellcurated LTR cohorts (Northwestern University - "NU" - Comprehensive Transplant Center Biorepository, and NIAID Clinical Trials in Organ Transplantation 14- CTOT- 14 NCT01672164), were analyzed to determine the role of dd-cfDNA in the detection of graft injury and rejection, particularly early signals in the setting of normal liver function tests in liver transplant recipients.

### Materials And Methods-Cohorts

Patient data for this study were collected from single center (NU:2010- 2015, 2019- 2020) and multicenter (CTOT14: 2012- 2014) cohort reported previously.

Briefly, the N U cohort consisted of adult deceased or live donor liver transplant recipients who had blood sample collected for biomarker studies only at the time of "for cause" graft biopsies (any time post- LT). The CTOT14 multicenter observational cohort enrolled adult deceased or live donor liver transplant recipients who were followed for 12- 24 months after transplant and had serial routine blood collections performed by study protocol as described below. All patient management (including immunosuppressive therapy regimens) were according to each center's standard of care and not mandated by study protocol. Written informed consent was obtained prospectively from each patient and the study protocols conformed to the ethical guidelines of the 1975 Declaration of Helsinki as reflected in a priori approval by the institution's human research committee. Inclusion criteria for both cohorts consisted of adult (≥18 years old) first transplant from either a deceased or living donor. Exclusion criteria were prior or multi-organ transplants, Human Immunodeficiency Virus infected, and viremic Hepatitis B and C patients.

Whole blood samples were collected at the time of "for cause" graft biopsies in both cohorts and serially at week 2, month 1, 2, 3, 6, 9, 12, 15, 18, 21, 24 months following LT in CTOT-14 only. All NU and CTOT- 14 biopsies were read locally for clinical care and later sent for independent, blinded central review using Banff criteria. Other causes of liver graft dysfunction (e.g., steatohepatitis, cholestasis, ischemia, etc.) were grouped together as acute dysfunction no rejection (ADNR). We also collected samples from liver transplant recipients (LTR) with longstanding normal liver tests (Clinical Phenotypes below) and labeled them "TX" as previous.

### Materials And Methods Clinical Phenotypes

Biopsy, biochemical and clinical criteria were used to define the following clinical diagnostic phenotypes: (1) AR: "for cause" biopsy consistent with AR and treated (treated biopsy- proven acute rejection); (2) ADNR: "for cause" biopsy consistent with a non-rejection etiology; (3) TX: normal LFTs at the time of and three months before and after sample collection: total bilirubin (TB) ≤1.5 mg/dl, alkaline phosphatase (AlkP) <200 U/L, and alanine transferase (ALT) ≤ 60 U/L (males), ≤ 36 U/L (females); (4) Non- AR: ADNR and TX combined, to evaluate all recipients without AR versus those with AR. For all prediagnosis (AR, ADNR, TX, Non- AR) serial samples (CTOT- 14 only), we required liver function tests to be normal at each pre-sample collection. For post- diagnosis (CTOT- 14 only), we focused only on dd- cfDNA changes following acute rejection therapy and normalization of liver function tests using the same criteria.

### Materials And Methods - Donor-derived Cell-free DNA Measurement

Blood for dd-cfDNA analysis was drawn in plasma separation tubes (BD Vacutainer^{®} PPT^{™} Plasma Preparation Tube, BD BioSciences) at the time of for cause biopsy, TX time point, and at serial pre-determined time points (CTOT- 14 only) prior to all phenotypes and after AR. Nucleic acid was purified by the QIAamp^{®} Circulating Nucleic Acid Kit with eluate dsDNA concentration determined using the Qubit 1X dsDNA HS Assay Kit (Invitrogen). NEBNext^{®} Ultra II library preparation was performed with an input of 0.5 ng/reaction. PCR amplification was performed using i7 and i5 primers followed by whole genome amplification enrichment. Library concentrations were determined using the Qubit 1X dsDNA HS Assay Kit, and size distributions were established using the Agilent 2200 or 4200 TapeStation^{®} and D1000 ScreenTape^{®}. Eight 4 nM libraries were pooled, with concentrations verified by Qubit analysis. Pooled 4 nM libraries were denatured, diluted to a final concentration of 1.2 pM (along with a PhiX Control library spiked in at 1% of the final concentration), and sequenced on the NextSeg^{®} 500 or 550 instrument using NextSeq^{®} Mid Output 500/550 flow cells and reagents and a 2 × 75 cycle paired-end sequencing protocol. Next-generation sequencing data were mapped to a human reference genome and, along with recipient genotype data (see below), analyzed for dd-cfDNA% by a bioinformatics pipeline licensed from Stanford University. Recipient genotyping was performed on PBMC samples collected from CPT tubes (BD Vacutainer^{®} CPT^{™} Mononuclear Cell Preparation Tube, BD BioSciences). Nucleic acid was purified using the Qiagen DNeasy^{®} Blood & Tissue kit. Hybridization to the Omni2.5- 8 BeadChip^{®} was performed. BeadChips^{®} are scanned on the Illumina iScan^{®} System with Illumina- provided DMAP files.

### Materials And Methods-Bioinformatics and Statistical DNA Analysis

Processed files were analyzed using the Illumina GenomeStudio^{®} (v.2.0.4) software in conjunction with a bead pool manifest file describing the single nucleotide polymorphisms contained in the bead pool used, and a cluster file containing cluster positions for each polymorphism needed to make genotype calls. dd-cfDNA results were provided as a percentage of the donor- derived fraction as compared to total cfDNA detected. Dd-cfDNA training set values were grouped by phenotype and receiver operator characteristic (ROC) curve analysis was performed on AR versus TX, AR versus ADNR and AR versus Non-AR values using GraphPad^{®} version 5.04.

Sensitivity/specificity (including 95% confidence intervals) and likelihood ratios were provided at continuous dd-cfDNA values for each analysis, and area under the curve (AUC) values were calculated by the Graphpad^{®} software. Youden index values were calculated as a general guideline for cutoff identification. However, the highest Yotidert index values were not strictly used to select the cutoff to differentiate phenotypes; rather dd-cfDNA cutoff values were selected which optimized both sensitivity and specificity values. The same ROC curves and AUC were generated for alanine aminotransferase (ALT) values alone or in combination with dd-cfDNA. To visualize the trend of dd-cfDNA scores, Linear Mixed Effect (LME) models were fitted for available samples up to 100 days surrounding the biopsy collection dates, including the biopsy time point. The models were then used to predict the values over this range.

### Results - Patient Cohorts

A total of 57 AR (43 NU, 14 CTOT- 14), 68 ADNR (41 NU, 27 CTOT-14), and 94 TX (47 NU, 47 CTOT- 14) patient samples were analyzed for dd-cfDNA levels at the diagnostic time points. The ADNR biopsies consisted of the following: 23 biliary obstruction/cholestasis, 16 non- specific minimal inflammation (not meeting any criteria for rejection), 14 steatosis, 6 ischemia/reperfusion injury, and 9 other causes. The groups were similar in terms of clinical characteristics, with the exception being a higher percentage of TX patients on tacrolimus therapy and differences in LFTs, as expected (Table 1).

**Table 1: Clinical Characteristics and Laboratory Values of Patient Phenotypes at Diagnosis for Participants in Studies Described Herein**

| | | **AR (N =57)** | **ADNR (N =68)** | **TX (N =94)** | ***p*** |
|---|---|---|---|---|---|
| **Age** | | 54.0 [33.8, 64.0] | 58.0 [46.0, 64.3] | 59.0 [49.8, 63.8] | 0.376^{b} |
| **Male sex (%)** | | 28 (49.1) | 41 (60.3) | 58 (61.7) | 0.284^{d} |
| **Race (%)** | | | | | 0.083^{c} |
| | **African American** | 10 (17.5) | 5 (7.4) | 3 ( 3.2) | |
| | **Hispanic/Latino** | 4 (7.0) | 5 (7.4) | 8 ( 8.5) | |
| | **Other** | 1 ( 1.8) | 5 (7.4) | 7 (7.4) | |
| | **White** | 42 (73.7) | 53 (77.9) | 76 (80.9) | |
| **Time from LT in months** | | 13.5 [5.90, 47.4] | 12.9 [4.8, 57.3] | 10.5 [6.0, 28.0] | 0.735^{b} |
| **Primary Diagnosis (%)** | | | | | 0.151^{c} |
| | **Alcohol** | 8 (14.0) | 10 (14.7) | 24 (25.5) | |
| | **Hepatitis C (nonviremic)** | 5 (8.8) | 4 (5.9) | 5 ( 5.3) | |
| | **Immune-Mediated (PSC, AIH, PBC)** | 15 (26.3) | 15 (22.1) | 19 (20.2) | |
| | **Non-Alcoholic** | 10 (17.5) | 22 (32.4) | 30 (31.9) | |
| **Fatty Liver or Cryptogenic** | | | | | |
| | **Other** | 19 (33.3) | 17 (25.0) | 16 (17.0) | |
| **Tacrolimus (%)** | | 45 (78.9) | 49 (72.1) | 84 (89.4) | **0.018^{d}** |
| **Mycophenolic acid (%)** | | 35 (61.4) | 31 (45.6) | 55 (58.5) | 0.146^{d} |
| **Alanine Aminotransferase (U/L)** | | 168.0 [89.0, 310.0] | 80.5 [40.5, 165.9] | 19.0 [12.3, 25.0] | **<0.001^{b}** |
| **Alkaline Phosphatase (U/L)** | | 199.0 [122.0, 295.0] | 171.0 [104.8, 309.8] | 75.5 [61.3, 96.0] | **<0.001^{b}** |
| **Total Bilirubin (mg/dL)** | | 1.10 [0.50, 2.2] | 0.9 [0.50, 3.7] | 0.60 [0.4, 0.8] | **<0.001^{b}** |
| **Creatinine (mg/dL)** | | 1.1 0 [0.91, 1.34] | 1.2 [0.94, 1.5] | 1.17 [1.0, 1.3] | 0.599^{b} |
| ^{a}All values expressed as either number (%) or median [IQR]. | | | | | |
| ^{b}p-values generated by Kruskal- Wallis rank sum test | | | | | |
| ^{c}p-values generated by Fisher's exact test. | | | | | |
| ^{d}p-values generated by Chi- square test. | | | | | |

| | | | | | |
|---|---|---|---|---|---|
| *Results-Dd-cfDNA at Time of Graff Dysfunction Versus Stable Function* | | | | | |

Median, inter- quartile dd- of DNA range and 5th- 95th percentile values are shown in Figure 1 and were statistically different between the groups (p < .0001). We also compared dd- cfDNA percentages with ALT values at diagnostic biopsy and found stronger correlations by Pearson coefficient in AR (r = .65, "moderate" positive correlation) versus ADNR (r = .32, "low" positive correlation) (p = .017).

The combined dd-cfDNA results at the time of diagnosis (N = 219) from each of the subgroups (AR, TX, ADNR) were then randomized 70:30 into training and test subsets, similar to gene expression study approaches that allow for accurate estimation while retaining a sufficient validation cohort to have confidence in the performance metrics. We also combined the populations because the cohorts had some differences (NU = single center, late post-liver transplant (post-LT); CTOT- 14 = multicenter, early post- LT) despite being phenotyped the same way clinically and histologically. This also allowed us to generate dd-cfDNA data that spanned both the early and late post-LT period.

Training set receiver operator characteristic (ROC) curve analyses for dd-cfDNA between AR versus TX and AR versus ADNR, as well as AR versus Non- AR, are displayed in Figure 2A- C. As an additional analysis, we analyzed ALT alone and in combination with dd-cfDNA in distinguishing all the phenotypes given the ALT differences at diagnosis from Table 1. All ROC curve analyses for dd-cfDNA alone were statistically significant, with higher AUC values for AR versus TX (0.95) compared to AR versus ADNR (0.71), and also AR versus Non- AR (AUC = 0.85). As shown in **Figure 2** panels A, B, and C, models using ALT alone or in combination with dd-cfDNA did not significantly enhance the AUC over dd-cfDNA alone. From the ROC curve analyses, dd-cfDNA cutoffs (AR vs. TX 5.3%; AR vs. ADNR 20.4%; AR vs. Non- AR 15%) were identified to optimize sensitivity and specificity for the comparisons. These cutoffs were applied to the test set with results shown in Table 2. Fisher's exact test (two-sided) was applied to each assessment with p values of <.001 (AR vs. TX), 0.049 (AR vs. ADNR), and <0.001 (AR vs. Non- AR).

**Table 2: Results of Test Set Analyses with Cutoff Values Established by Training Samples**

| | | **Number** of **Samples** | | | | | | **Adjusted^{a}** | | |
|---|---|---|---|---|---|---|---|---|---|---|
| **Category** | **dd-cfDNA cutoff** | **TP** | **FP** | **TN** | **FN** | **Sens.** | **Spec.** | **PPV** | **NPV** | ***p* value** |
| **AR vs TX** | 5.3% | 16 | 6 | 24 | 0 | 100% | 80% | 62.5% | 100% | <0.001 |
| **AR vs ADNR** | 20.4% | 12 | 8 | 12 | 4 | 75% | 60% | 38.5% | 87.8% | 0.049 |
| **AR vs Non-AR** | 15.0% | 14 | 10 | 40 | 2 | 87.5% | 80% | 58.3% | 95.1% | <0.001 |
| Abbreviations: FN, false negative; FP, false positive; NPV, negative predictive value; PPV, positive predictive value; TN, true negative; TP, true positive. | | | | | | | | | | |
| ^{a}Adjusted to 25% prevalence of AR and 25% prevalence of ANDR (if applicable). | | | | | | | | | | |

### Results-Serial dd-cfDNA before and after graft dysfunction vs stable function

We focused on the 100 days before and at the AR, ADNR, TX diagnostic time points to have more proximity of dd- cfDNA values to the events, as well as after AR diagnosis. LME model- derived dd-cfDNA values, along with 95% confidence interval of error zones, were plotted over time **(****Figure 3** panels A, B, and C). In CTOT- 14, 195 samples (33 AR, 32 ADNR and 130 TX) for dd-cfDNA were collected serially in stable patients (normal LFTs at each collection). Of these, 60 (12 AR, 8 ADNR, 40 TX) samples fell within 100 days prior to the biopsy time point and 88 (14 AR, 27 ADNR, 47 TX) at the biopsy time point- all included in the serial analysis and LME model. Thus, 148 samples were profiled for dd- cfDNA in 88 LTR (14 AR, 27 ADNR, 47 TX). Thirty- six additional samples were collected following treatment and laboratory resolution of the AR cases, 21 within 100 days.

**Figure 3** panel A shows a steady increase in dd- cfDNA prior to both AR and ADNR (above the cutoffs reported above) and no change serially over time in TX (below the cutoff). The increase was greater and steeper for AR compared to ADNR. Importantly, there was no increase in liver function tests (ALT or AlkP) over time, even within the normal range, in this pre-diagnosis period (**Figure 4****).** As shown, at the time point (day-48) where dd-cfDNA levels increased above the AR versus ADNR cut- off of 20.4%, ALT and AlkP levels were in the normal range and not statistically different between AR and ADNR (42.7 vs. 32.4 U/L, 87.8 vs. 91.5 U/L, respectively). **Figure 3** panel B shows an additional comparison of AR versus Non- AR, distinguishing the serial dd-cfDNA trajectory leading up to AR from all other phenotypes. Following the diagnosis and treatment of AR, dd- cfDNA decreased alongside normalization of liver function tests (**Figure 3** panel C).

### Discussion

Quantification of dd-cfDNA in plasma uses allelic variations present in organ donor versus transplant recipient genes to estimate the fraction of circulating dd-cfDNA. Genome sequencing allows precise, reproducible quantification of the graft and recipient cell-free DNA fractions due to a large number of polymorphic allelic loci allowing for the expression of a percentage of donor relative to recipient cell-free DNA. While dd-cfDNA is present at low levels in the plasma of all recipients, graft injury can cause increase in the number of donor relative to recipient DNA molecules, facilitating identification of graft injury .

The aims of the current study were: (1) to orthogonally validate the clinical utility of a dd-cfDNA assay in the diagnosis of graft dysfunction (elevated LFTs) in liver transplant rejection using a dd-cfDNA platform, (2) to test the ability of this assay to differentiate between rejection and other causes of graft dysfunction, and (3) to serially detect early graft injury in the setting of normal LFTs, prior to the onset of elevated LFTs and graft dysfunction. To achieve these aims, patient cohorts that included graft dysfunction phenotypes that included both AR and ADNR were utilized, as well as a normal function phenotype (TX). Additionally, serial samples with normal LFTs prior to AR, ADNR, and TX, as well as following treatment of AR, were analyzed, allowing changes within individual patient context to be observed.

The results demonstrated that dd-cfDNA levels can differentiate between the AR versus TX phenotypes (5.3% cutoff) and the AR versus ADNR phenotypes (20.4% cutoff). There was a stronger R-value correlation between liver test values and rejection versus non-rejection causes at diagnosis, which essentially reflects more direct graft inflammation/injury in rejection. Using these cutoff values in the test set, the accuracy and negative predictive value of AR versus TX was 87% and 100%, and 66.7% and 87.8% for AR versus ADNR. Additional analyses showed that a 15% dd-cfDNA cutoff can distinguish AR from all other phenotypes, regardless of LFTs at diagnosis. Thus, differences in dd-cfDNA between AR, other causes, and stable function in liver transplant recipients were validated. Moreover, in utilizing both single center and NIAID CTOT-14 multicenter cohorts, the current study includes a significantly larger sample size and thus allowed for adequate discovery (training) and validation (test) analyses required for clinical utility.

In addition, using prospectively collected serial samples linked to clinical data from the CTOT-14 cohort, dd-cfDNA levels over time leading up to graft dysfunction, manifested by elevated LFTs and for- cause biopsies demonstrating AR versus ADNR were longitudinally evaluated. Serial samples in stable LTR (TX) over time as a comparison were also evaluated. Blood samples preceding the onset of either AR or ADNR showed elevations in dd-cfDNA above the validated cut-off values and consistently below in TX. These preceding samples were all collected at time points where LFTs were in the normal range, and thus dd-cfDNA appears to be sensitively detecting graft injury occurring before the increase in LFTs. Importantly, the dd-cfDNA levels were higher with a steeper trajectory preceding AR than ADNR, as well as versus all other LTR (Non- AR), suggesting dd-cfDNA is released from the graft to the greatest degree preceding AR (which may be helpful in immunosuppressant tapering decisions). The current study also demonstrated that dd-cfDNA levels decline following treatment of AR and normalization of LFTs. This appears to eb the first report that demonstrates elevations of dd-cfDNA in stable LTR with normal LFTs prior to the onset of laboratory evidence of graft dysfunction, as well as reduction of dd-cfDNA following resolution of AR. These novel data strongly suggest a putative role for serial determinations of dd-cfDNA that could be used for monitoring LTR, allowing clinicians to detect early signs of AR in stable patients, perhaps in the setting of IS minimization.

These findings are of particular interest given the need for biomarkers that can detect immune activation versus quiescence in transplant recipients in general. While current immunosuppression regimens are generally effective in preventing and treating acute rejection (AR), their associated complications negatively affect their overall effectiveness. As a result, there is a strong interest in immunosuppression minimization protocols to both prevent and treat immunosuppression complications, including nephrotoxicity due to CNI therapy among others. Yet without specific tests indicating the onset of immune activation, clinicians currently perform arbitrary 'trial and error' immunosuppression reductions which can trigger rejection and alternatively the need for higher prolonged immunosuppressive (IS) dosing and worse overall outcomes. To mitigate this risk, non-invasive biomarkers that can signal immune activation and imminent graft dysfunction in the context of immunosuppression tapering would be a welcome addition to liver transplant recipient management.

Given the performance indicated for the current dd-cfDNA test, an additional question would be whether the test may complement existing methodologies for detecting AR in addition to being used in a standalone fashion. However, the current data indicates this test does have advantages over standard liver enzymes in detecting early signs of graft injury and rejection in serial monitoring, particularly given the rise in dd-cfDNA occurring prior to alanine aminotransferase (ALT) elevation, as well as the finding herein that finding that ALT alone, when elevated, may be insufficient in distinguishing rejection from other causes.

In conclusion, the current study has provided validation that elevated dd-cfDNA represents a clear signal of early graft injury in LTR and is most prominent in AR than ADNR. A key associated finding is that the proportion of dd-cfDNA increases serially preceding elevation of LFTs and clinically evident graft dysfunction and decreases serially following resolution of AR. These data suggest a putative role for this novel biomarker for serial monitoring of LTR, particularly in the context of immunosuppression minimization strategies.

While certain exemplary embodiments of the present invention have been shown and described herein, it will be obvious to those skilled in the art that such embodiments are provided by way of example only. Numerous variations, changes, and substitutions will now occur to those skilled in the art. It should be understood that various alternatives to the embodiments of the invention described herein may be employed in practicing the invention. It is intended that the following claims define the scope of the invention and that methods and structures within the scope of these claims be covered thereby.

## Claims

1. A method of distinguishing non-rejection (TX) from one or both of acute rejection (AR) and acute dysfunction non-rejection (ADNR) in a liver transplant recipient, the method comprising:
(a) obtaining cell-free DNA (cfDNA) from a sample obtained from a liver transplant recipient;
(b) determining a level of donor derived cell-free DNA (dd-cfDNA) in the cfDNA; and
(c) distinguishing non-rejection from one or both of AR and ADNR in the recipient by comparing the level of dd-cfDNA to a pre-determined threshold value,
wherein the recipient is indicated as having AR or ADNR by a level of dd-cfDNA that is greater than or equal to a pre-determined threshold of from 10% to 17%, from 13% to 17%, from 13% to 15%, from 15% to 17%, from 14% to 17%, from 14% to 16%, from 14% to 15%, from 15% to 16%, from 14.5% to 15.5%, or a pre-determined threshold of 10%, 11%, 12%, 13%, 14%, 14.5%, 15%, 15.5%, 16%, or 17%.

2. The method of claim 1, wherein the level of dd-cfDNA is determined by sequencing the cfDNA, and inputting into a computer-implemented algorithm (i) cfDNA sequence information at a plurality of genetic loci, and (ii) genotype information from the recipient, in the absence of genotype information from the donor, wherein the algorithm identifies dd-cfDNA sequences at the plurality of genetic loci by identifying a pattern of cfDNA sequences at the loci that differ from the cfDNA sequences of the recipient but that correlate with a pattern of human DNA sequences found in a database of human genomes.

3. The method of claim 1 or 2, wherein determining the level of dd-cfDNA comprises analyzing cfDNA sequences of at least 1000, at least 2000, at least 5000, at least 10,000, at least 20,000, at least 100,000, at least 500,000, at least 1,000,000, at least 2,000,000, or at least 2,500,000 genetic loci, such as SNPs.

4. The method of any one of claims 1-3, wherein the sample is a blood, serum, plasma, or urine sample, optionally wherein the sample is a blood sample, optionally wherein plasma is separated from the blood sample and/or wherein peripheral blood mononuclear cells (PBMCs) are isolated from the blood sample.

5. The method of claim 4, wherein PBMCs are isolated from the blood sample and wherein genotype information from the recipient is obtained from the PBMCs.

6. The method of any one of claims 1-5, wherein
(a) said liver transplant recipient has a normal serum alanine aminotransferase concentration, such as of less than or equal to 60 IU/L for a male recipient or less than or equal to 36 IU/L for a female recipient, or wherein the serum alanine aminotransferase concentration has not doubled compared to a baseline concentration determined after transplantation, (b) said liver transplant recipient has a normal serum aspartate aminotransferase concentration, such as below 40 IU/L, or wherein the serum aspartate aminotransferase concentration has not doubled compared to a baseline concentration determined after transplantation, (c) said liver transplant recipient has a normal serum alkaline phosphatase level, such as below 200 IU/L, or wherein the serum alkaline phosphatase level has not doubled compared to a baseline concentration determined after transplantation, (d) said liver transplant recipient has a normal total bilirubin concentration, such as less than or equal to 1.5 mg/dL, or wherein the total bilirubin concentration has not doubled compared to a baseline concentration determined after transplantation, or (e) wherein said liver transplant recipient has a normal direct bilirubin concentration, such as below 0.5 mg/dL, or wherein the direct bilirubin concentration has not doubled compared to a baseline concentration determined after transplantation.

7. The method of any one of claims 1-6, wherein the determining the level of the dd-cfDNA comprises sequencing the cfDNA by whole genome sequencing.

8. The method of any one of claims 1-7, further wherein the recipient is indicated as having AR by a level of dd-cfDNA that is greater than or equal to a pre-determined threshold of from 18% to 25%, from 18% to 23%, from 19% to 22%, from 19% to 21%, from 20% to 22%, from 20% to 21%, from 20.2% to 20.6%, or a pre-determined threshold of 18%, 19%, 20%, 20.2%, 20.4%, 20.6%, 20.8%, 21%, 22%, 23%, or 25%.

9. The method of any one of claims 1-7, wherein the recipient is indicated as having AR by a level of dd-cfDNA that is greater than or equal to a pre-determined threshold of 20.4%.

10. The method of any one of claims 1-8, wherein the recipient is indicated as having AR or ADNR by a level of dd-cfDNA that is greater than or equal to a pre-determined threshold of 15.0%.

11. The method of any one of claims 1-10, further wherein the recipient is indicated as having non-rejection by a level of dd-cfDNA that is less than a pre-determined threshold of from 4% to 7%, from 4% to 6%, from 5% to 7%, from 4.5% to 6.5%, from 4.5% to 5.5%, from 5% to 6%, from 5% to 5.5%, or less than a pre-determined threshold value of 4%, 4.5%, 5%, 5.1%, 5.2%, 5.3%, 5.4%, 5.5%, 6%, 6.5%, or 7%.

12. The method of any one of claims 1-10, wherein the recipient is indicated as having non-rejection by a level of dd-cfDNA that is less than a pre-determined threshold of 5.3%.

13. The method of any one of claims 1-12, wherein the recipient is indicated as having ADNR by a level of dd-cfDNA that is greater than or equal to a pre-determined threshold of 5.3% but less than a pre-determined threshold of 15.0%.

14. The method of any one of claims 1-13, wherein the method is performed at least one month, at least two months, at least three months, at least six months, or at least one year after transplantation.

15. The method of any one of claims 1-14, wherein the method further comprises determining one or more of: alanine aminotransferase concentration, aspartate aminotransferase concentration, alkaline phosphatase concentration, direct bilirubin concentration, or bilirubin concentration in the sample,
, wherein the method further comprises providing a report summarizing the recipient's level of dd-cfDNA, and optionally one or more potential treatments for the recipient, and/or wherein the method further comprises selecting a treatment for the recipient based on the recipient's level of dd-cfDNA.

## Patentansprüche

1. Verfahren zum Unterscheiden von Nicht-Abstoßung (TX) von einer oder beiden von akuter Abstoßung (AR) und von akuter Dysfunktion ohne Abstoßung (ADNR) in einem Lebertransplantatempfänger, das Verfahren umfassend:
(a) Erhalten zellfreier DNA (cfDNA) aus einer Probe, die von einem Lebertransplantatempfänger erhalten wird;
(b) Bestimmen eines Spiegels spenderstammender zellfreier DNA (dd-cfDNA) in der cfDNA; und
(c) Unterscheiden von Nicht-Abstoßung von einer oder beiden von AR und ADNR in dem Empfänger durch Vergleichen des Spiegels von dd-cfDNA mit einem zuvor bestimmten Schwellenwert, wobei der Empfänger als AR oder ADNR aufweisend durch einen Spiegel von dd-cfDNA, der größer als oder gleich einem zuvor bestimmten Schwellenwert von 10 % bis 17 %, von 13 % bis 17 %, von 13 % bis 15 %, von 15 % bis 17 %, von 14 % bis 17 %, von 14 % bis 16 %, von 14 % bis 15 %, von 15 % bis 16 %, von 14,5 % bis 15,5 %, oder einem zuvor bestimmten Schwellenwert von 10 %, 11 %, 12 %, 13 %, 14 %, 14,5 %, 15 %, 15,5 %, 16 %, oder 17 % ist, angezeigt wird.

2. Verfahren nach Anspruch 1, wobei der Spiegel von dd-cfDNA bestimmt wird durch Sequenzierung der cfDNA und Eingeben in einen computerimplementierten Algorithmus von (i) cfDNA-Sequenzinformationen an einer Vielzahl von genetischen Loci und (ii) Genotypinformationen von dem Empfänger, in der Abwesenheit von Genotypinformationen von dem Spender, wobei der Algorithmus dd-cfDNA-Sequenzen an der Vielzahl von genetischen Loci durch Identifizieren eines Musters von cfDNA-Sequenzen an den Loci identifiziert, die sich von den cfDNA-Sequenzen des Empfängers verschieden sind, die aber mit einem Muster menschlicher DNA-Sequenzen, die in einer Datenbank menschlicher Genome gefunden werden, korrelieren.

3. Verfahren nach Anspruch 1 oder 2, wobei das Bestimmen des Spiegels von dd-cfDNA ein Analysieren von cfDNA-Sequenzen von mindestens 1000, mindestens 2000, mindestens 5000, mindestens 10.000, mindestens 20.000, mindestens 100.000, mindestens 500.000, mindestens 1.000.000, mindestens 2.000.000 oder mindestens 2.500.000 genetischen Loci, wie SNPs, umfasst.

4. Verfahren nach einem der Ansprüche 1 bis 3, wobei die Probe eine Blut-, Serum-, Plasma- oder Urinprobe ist, optional wobei die Probe eine Blutprobe ist, optional wobei Plasma von der Blutprobe getrennt wird, und/oder wobei mononukleäre Zellen peripheren Blutes (PBMCs) aus der Blutprobe isoliert werden.

5. Verfahren nach Anspruch 4, wobei PBMCs aus der Blutprobe isoliert werden, und wobei Genotypinformationen von dem Empfänger aus den PBMCs erhalten werden.

6. Verfahren nach einem der Ansprüche 1 bis 5, wobei
(a) der Lebertransplantatempfänger eine normale Serum-Alanin-Aminotransferase-Konzentration, wie von weniger als oder gleich 60 IU/L für einen männlichen Empfänger oder weniger als oder gleich 36 IU/L für einen weiblichen Empfänger, aufweist, oder wobei sich die Serum-Alanin-Aminotransferase-Konzentration verglichen mit einer Baseline-Konzentration, die nach Transplantation bestimmt wird, nicht verdoppelt hat, (b) der Lebertransplantatempfänger eine normale Serum-Aspartat-Aminotransferase-Konzentration, wie unter 40 IU/L, aufweist, oder wobei sich die Serum-Aspartat-Aminotransferase-Konzentration verglichen mit einer Baseline-Konzentration, die nach Transplantation bestimmt wird, nicht verdoppelt hat, (c) der Lebertransplantatempfänger einen normalen Serum-alkalische-Phosphatase-Spiegel, wie unter 200 IU/L, aufweist oder wobei sich der Serum-Alkalische-Phosphatase-Spiegel verglichen mit einer Baseline-Konzentration, die nach Transplantation bestimmt wird, nicht verdoppelt hat, (d) der Lebertransplantatempfänger eine normale Gesamt-Bilirubin-Konzentration, wie weniger als oder gleich 1,5 mg/dL, aufweist, oder wobei sich die Gesamt-Bilirubin-Konzentration verglichen mit einer Baseline-Konzentration, die nach Transplantation bestimmt wird, nicht verdoppelt hat, oder (e) wobei der Lebertransplantatempfänger eine normale direkte Bilirubin-Konzentration, wie unter 0,5 mg/dL, aufweist, oder wobei sich die direkte Bilirubin-Konzentration verglichen mit einer Baseline-Konzentration, die nach Transplantation bestimmt wird, nicht verdoppelt hat.

7. Verfahren nach einem der Ansprüche 1 bis 6, wobei das Bestimmen des Spiegels der dd-cfDNA das Sequenzieren der cfDNA durch Gesamtgenomsequenzierung umfasst.

8. Verfahren nach einem der Ansprüche 1 bis 7, wobei ferner der Empfänger als AR aufweisend durch einen Spiegel von dd-cfDNA, der größer als oder gleich einem zuvor bestimmten Schwellenwert von 18 % bis 25 %, von 18 % bis 23 %, von 19 % bis 22 %, von 19 % bis 21 %, von 20 % bis 22 %, von 20 % bis 21 %, von 20,2 % bis 20,6 %, oder einem zuvor bestimmten Schwellenwert von 18 %, 19 %, 20 %, 20,2 %, 20,4 %, 20,6 %, 20,8 %, 21 %, 22 %, 23 % oder 25 % ist, angezeigt wird.

9. Verfahren nach einem der Ansprüche 1 bis 7, wobei der Empfänger als AR aufweisend durch einen Spiegel von dd-cfDNA, der größer als oder gleich einem zuvor bestimmten Schwellenwert von 20,4 % ist, angezeigt wird.

10. Verfahren nach einem der Ansprüche 1 bis 8, wobei der Empfänger als AR oder ADNR aufweisend durch einen Spiegel von dd-cfDNA, der größer als oder gleich einem zuvor bestimmten Schwellenwert von 15,0 % ist, angezeigt wird.

11. Verfahren nach einem der Ansprüche 1 bis 10, wobei ferner der Empfänger als Nicht-Abstoßung aufweisend durch einen Spiegel von dd-cfDNA, der weniger als ein zuvor bestimmter Schwellenwert von 4 % bis 7 %, von 4 % bis 6 %, von 5 % bis 7 %, von 4,5 % bis 6,5 %, von 4,5 % bis 5,5 %, von 5 % bis 6 %, von 5 % bis 5,5 %, oder weniger als ein zuvor bestimmter Schwellenwert von 4 %, 4,5 %, 5 %, 5,1 %, 5,2 %, 5,3 %, 5,4 %, 5,5 %, 6 %, 6,5 % oder 7 % ist, angezeigt wird.

12. Verfahren nach einem der Ansprüche 1 bis 10, wobei der Empfänger als Nicht-Abstoßung aufweisend durch einen Spiegel von dd-cfDNA, der weniger als ein zuvor bestimmter Schwellenwert von 5,3 % ist, angezeigt wird.

13. Verfahren nach einem der Ansprüche 1 bis 12, wobei der Empfänger als ADNR aufweisend durch einen Spiegel von dd-cfDNA, der größer als oder gleich einem zuvor bestimmten Schwellenwert von 5,3 %, aber weniger als ein zuvor bestimmter Schwellenwert von 15,0 % ist, angezeigt wird.

14. Verfahren nach einem der Ansprüche 1 bis 13, wobei das Verfahren mindestens einen Monat, mindestens zwei Monate, mindestens drei Monate, mindestens sechs Monate oder mindestens ein Jahr nach Transplantation durchgeführt wird.

15. Verfahren nach einem der Ansprüche 1 bis 14, wobei das Verfahren ferner das Bestimmen einer oder mehrerer umfasst von: Alanin-Aminotransferase-Konzentration, Aspartat-Aminotransferase-Konzentration, alkalische Phosphatase-Konzentration, direkter Bilirubin-Konzentration oder Bilirubin-Konzentration in der Probe,
wobei das Verfahren ferner ein Bereitstellen eines Berichts, der den Spiegel von dd-cfDNA des Empfängers zusammenfasst, und optional eine oder mehrere potentielle Behandlungen für den Empfänger, umfasst, und/oder wobei das Verfahren ferner ein Auswählen einer Behandlung für den Empfänger basierend auf dem Spiegel von dd-cfDNA des Empfängers umfasst.

## Revendications

1. Procédé de distinction d'une absence de rejet (TX) par rapport à l'un et/ou l'autre d'un rejet aigu (AR) et d'un dysfonctionnement aigu sans rejet (ADNR) chez un receveur de greffe de foie, le procédé comprenant :
(a) l'obtention d'ADN acellulaire (ADNac) auprès d'un échantillon obtenu auprès d'un receveur de greffe de foie ;
(b) la détermination d'un taux d'ADN acellulaire dérivé du donneur (dd-ADNac) dans l'ADNac ; et
(c) la distinction d'une absence de rejet par rapport à l'un et/ou l'autre d'un AR et d'un ADNR chez le receveur en comparant le taux de dd-ADNac à une valeur seuil prédéterminée, dans lequel le receveur est indiqué comme ayant un AR ou un ADNR par un taux de dd-ADNac qui est supérieur ou égal à un seuil prédéterminé allant de 10 % à 17 %, de 13 % à 17 %, de 13 % à 15 %, de 15 % à 17 %, de 14 % à 17 %, de 14 % à 16 %, de 14 % à 15 %, de 15 % à 16 %, de 14,5 % à 15,5 %, ou un seuil prédéterminé de 10 %, 11 %, 12 %, 13 %, 14 %, 14,5 %, 15 %, 15,5 %, 16 % ou 17 %.

2. Procédé selon la revendication 1, dans lequel le taux de dd-ADNac est déterminé par séquençage de l'ADNac, et entrée dans un algorithme implémenté par ordinateur (i) d'informations de séquence d'ADNac au niveau d'une pluralité de loci génétiques, et (ii) d'informations génotypiques provenant du receveur, en l'absence d'informations génotypiques provenant du donneur, dans lequel l'algorithme identifie des séquences de dd-ADNac au niveau de la pluralité de loci génétiques en identifiant un motif de séquences d'ADNac au niveau des loci qui diffèrent des séquences d'ADNac du receveur, mais qui sont en corrélation avec un motif de séquences d'ADN humaines trouvé dans une base de données de génomes humains.

3. Procédé selon la revendication 1 ou 2, dans lequel la détermination du taux de dd-ADNac comprend l'analyse de séquences d'ADNac d'au moins 1000, d'au moins 2000, d'au moins 5000, d'au moins 10 000, d'au moins 20 000, d'au moins 100 000, d'au moins 500 000, d'au moins 1 000 000, d'au moins 2 000 000, ou d'au moins 2 500 000 loci génétiques, tels que des SNP.

4. Procédé selon l'une quelconque des revendications 1 à 3, dans lequel l'échantillon est un échantillon de sang, de sérum, de plasma ou d'urine, facultativement dans lequel l'échantillon est un échantillon de sang, facultativement dans lequel le plasma est séparé de l'échantillon de sang et/ou dans lequel des cellules mononucléaires de sang périphérique (PBMC) sont isolées de l'échantillon de sang.

5. Procédé selon la revendication 4, dans lequel des PBMC sont isolés de l'échantillon de sang et dans lequel des informations génotypiques provenant du receveur sont obtenues par les PBMC.

6. Procédé selon l'une quelconque des revendications 1 à 5, dans lequel
(a) ledit receveur de greffe de foie a une concentration normale en alanine aminotransférase sérique, telle qu'inférieure ou égale à 60 IU/L pour un receveur mâle ou inférieure ou égale à 36 IU/L pour un receveur femelle, ou dans lequel la concentration en alanine aminotransférase sérique n'a pas doublé par comparaison avec une concentration de ligne de base déterminée après transplantation, (b) ledit receveur de greffe de foie a une concentration normale en aspartate aminotransférase sérique, telle qu'inférieure à 40 IU/L, ou dans lequel la concentration en aspartate aminotransférase sérique n'a pas doublé par comparaison avec une concentration de ligne de base déterminée après transplantation, (c) ledit receveur de greffe de foie a un taux normal en phosphatase alcaline sérique, tel qu'inférieur à 200 IU/L, ou dans lequel le taux en phosphatase alcaline sérique n'a pas doublé par comparaison avec une concentration de ligne de base déterminée après transplantation, (d) ledit receveur de greffe de foie a une concentration normale en bilirubine totale, telle qu'inférieure ou égale à 1,5 mg/dL, ou dans lequel la concentration en bilirubine totale n'a pas doublé par comparaison avec une concentration de ligne de base déterminée après transplantation, ou (e) dans lequel ledit receveur de greffe de foie a une concentration normale en bilirubine directe, telle qu'inférieure à 0,5 mg/dL, ou dans lequel la concentration en bilirubine directe n'a pas doublé par comparaison avec une concentration de ligne de base déterminée après transplantation.

7. Procédé selon l'une quelconque des revendications 1 à 6, dans lequel la détermination du taux du dd-ADNac comprend le séquençage de l'ADNac par séquençage génomique complet.

8. Procédé selon l'une quelconque des revendications 1 à 7, dans lequel en outre le receveur est indiqué comme ayant un AR par un taux de dd-ADNac qui est supérieur ou égal à un seuil prédéterminé allant de 18 % à 25 %, de 18 % à 23 %, de 19 % à 22 %, de 19 % à 21 %, de 20 % à 22 %, de 20 % à 21 %, de 20,2 % à 20,6 %, ou un seuil prédéterminé de 18 %, 19 %, 20 %, 20,2 %, 20,4 %, 20,6 %, 20,8 %, 21 %, 22 %, 23 % ou 25 %.

9. Procédé selon l'une quelconque des revendications 1 à 7, dans lequel le receveur est indiqué comme ayant un AR par un taux de dd-ADNac qui est supérieur ou égal à un seuil prédéterminé de 20,4 %.

10. Procédé selon l'une quelconque des revendications 1 à 8, dans lequel le receveur est indiqué comme ayant un AR ou un ADBR par un taux de dd-ADNac qui est supérieur ou égal à un seuil prédéterminé de 15,0 %.

11. Procédé selon l'une quelconque des revendications 1 à 10, dans lequel en outre le receveur est indiqué comme ayant une absence de rejet par un taux de dd-ADNac qui est inférieur à un seuil prédéterminé allant de 4 % à 7 %, de 4 % à 6 %, de 5 % à 7 %, de 4,5 % à 6,5 %, de 4,5 % à 5,5 %, de 5 % à 6 %, de 5 % à 5,5 %, ou inférieur à une valeur seuil prédéterminée de 4 %, 4,5 %, 5 %, 5,1 %, 5,2 %, 5,3 %, 5,4 %, 5,5 %, 6 %, 6,5 % ou 7 %.

12. Procédé selon l'une quelconque des revendications 1 à 10, dans lequel le receveur est indiqué comme ayant une absence de rejet par un taux de dd-ADNac qui est inférieur à un seuil prédéterminé de 5,3 %.

13. Procédé selon l'une quelconque des revendications 1 à 12, dans lequel le receveur est indiqué comme ayant un ADNR par un taux de dd-ADNac qui est supérieur ou égal à un seuil prédéterminé de 5,3 % mais inférieur à un seuil prédéterminé de 15,0 %.

14. Procédé selon l'une quelconque des revendications 1 à 13, dans lequel le procédé est mis en œuvre au moins un mois, au moins deux mois, au moins trois mois, au moins six mois, ou au moins un an après transplantation.

15. Procédé selon l'une quelconque des revendications 1 à 14, dans lequel le procédé comprend en outre la détermination d'une ou plusieurs parmi :
concentration en alanine aminotransférase, concentration en aspartate aminotransférase, concentration en phosphatase alcaline, concentration en bilirubine directe ou concentration en bilirubine dans l'échantillon,
, dans lequel le procédé comprend en outre la fourniture d'un rapport résumant le taux de dd-ADNac du receveur, et facultativement un ou plusieurs traitements potentiels pour le receveur, et/ou dans lequel le procédé comprend en outre la sélection d'un traitement pour le receveur en fonction du taux de dd-ADNac du receveur.
